# EUROPEAN PATENT SPECIFICATION

(11) **EP 2 569 453 B1**
(45) Date of publication and mention of the grant of the patent: **16.12.2015**
(21) Application number: 11781412.9
(22) Date of filing: 16.05.2011
(51) Int. Cl.: C12Q 1/68, C12N 15/10

(54) **NUCLEIC ACID ISOLATION METHODS**
NUKLEINSÄURE-ISOLATIONSVERFAHREN
MÉTHODES D'ISOLEMENT DE L'ACIDE NUCLÉIQUE

(30) Priority: 14.05.2010 US 345063 P
(43) Date of publication of application: 20.03.2013
(73) Proprietor: Fluidigm Corporation, South San Francisco, CA 94080 (US)
(72) Inventor: MAY, Andrew, San Francisco California 94121 (US); MIR, Alain, Cupertino California 95014 (US); RAMAKRISHNAN, Ramesh, San Jose California 95120 (US); ZIMMERMANN, Bernhard, San Mateo California 94403 (US)
(74) Representative: Patent Boutique LLP
(86) International application number: PCT/US2011/036670
(87) International publication number: WO 2011/143659

(56) References cited:
- WO-A2-2009/032779
- US-A1- 2002 106 686
- US-A1- 2009 099 041
- US-A1- 2010 120 098
- US-A1- 2010 121 044
- LI YING ET AL: "Size separation of circulatory DNA in maternal plasma permits ready detection of fetal DNA polymorphisms", CLINICAL CHEMISTRY, AMERICAN ASSOCIATION FOR CLINICAL CHEMISTRY, WASHINGTON, DC, vol. 50, no. 6, 8 April 2004 (2004-04-08), pages 1002-1011, XP002510472, ISSN: 0009-9147, DOI: 10.1373/CLINCHEM.2003.029835
- A. SIKORA ET AL: "Detection of Increased Amounts of Cell-Free Fetal DNA with Short PCR Amplicons", CLINICAL CHEMISTRY, vol. 56, no. 1, 2 November 2009 (2009-11-02), pages 136-138, XP055081573, ISSN: 0009-9147, DOI: 10.1373/clinchem.2009.132951
- GUO, D. ET AL.: 'Methodology for using a universal primer to label amplified DNA segments for molecular analysis' BIOTECHNOLOGY LETTERS. vol. 25, no. 24, December 2003, pages 2079 - 2083
- POLIDOROS, A. N. ET AL.: ''Rolling circle amplification-RACE: a method for simultaneous isolation of 5' and 3' cDNA ends from amplified cDNA templates'' BIOTECHNIQUES. vol. 41, no. 1, July 2006, page 35, 36, 38, 40
- SCHAEFER, B. C. ET AL.: 'Revolutions in rapid amplification of cDNA ends: new strategies for polymerase chain reaction cloning of full-length cDNA ends' ANALYTICAL BIOCHEMISTRY. vol. 227, no. 2, 20 May 1995, pages 255 - 273

## Description

### CROSS-REFERENCES TO RELATED APPLICATIONS

This application claims the benefit of U.S. Provisional Application No. 61/345,063, filed on May 14, 2010.

### FIELD OF THE INVENTION

The present invention relates to generally to the area of selectively enriching a biological sample for short (e.g., less than 300 nucleotides), as opposed to long nucleic acids.

### BACKGROUND OF THE INVENTION

Cell-free fetal DNA is present in maternal bodily fluids from a pregnant woman, such blood, cervico-vaginal secretions, urine, etc. Detecting genotype (e.g., mutations) and/or aneuploidy in such fetal DNA in a maternal sample is difficult due to the presence of cell-free maternal DNA at a much higher percentage than the fetal DNA, which constitutes only about 5 percent, or less, of the total DNA in such samples. Similar difficulties exist with respect to the detection of cell-free tumor DNA in bodily fluids from cancer patients.

### SUMMARY OF THE INVENTION

The invention provides methods of enriching a sample for short nucleic acids of less than 300 nucleotides in length. The methods comprise:
a) circularizing the sample nucleic acids under conditions that favor the circularization of short nucleic acids; and
b) recovering the circularized nucleic acids. In some embodiments, the circularizing is carried out by contacting the sample nucleic acids with a circligase.

In some embodiments, any of the above-described methods further comprise the step of nucleic acids amplification and/or DNA sequencing to detect and/or quantify target nucleic acids within the short nucleic acids.

In some embodiments of these methods, the sample is unfractionated prior to enrichment. In some embodiments, the sample comprises a sample of a bodily fluid, or a fraction thereof, from a cancer patient. In some embodiments, the sample comprises a sample of a maternal bodily fluid, or a fraction thereof, from a pregnant subject. In some embodiments, the maternal bodily fluid is selected from the group consisting of whole blood, plasma, urine, and cervico-vaginal secretions.

In some embodiments, at least some of the short nucleic acids comprise fetal DNA. In some embodiments, the methods comprise determining a fetal genotype. In some embodiments, the methods comprise detecting the presence of a mutation or fetal aneuploidy.

### BRIEF DESCRIPTION OF THE DRAWINGS

Figure 1 illustrates that when free transposon ends are used in a reaction, the target DNA is fragmented and the transferred strand of the transposon end oligonucleotide is covalently attached to the 5'end of the fragment.
Figure 2 illustrates that high molecular weight DNA is selectively precipitated using PEG 8000 and NaCl; following centrifugation, small nucleic acid-apopototic-length DNAs are present in the supernatant.

### DETAILED DESCRIPTION

The present invention provides methods for selectively enriching a biological sample for short nucleic acids, such as fetal DNA in a maternal sample or apoptic DNA in a biological sample from a cancer patient and for subsequently analyzing the short nucleic acids for genotype, mutation, and/or aneuploidy.

### Definitions

Terms used in the claims and specification are defined as set forth below unless otherwise specified. These terms are defined specifically for clarity, but all of the definitions are consistent with how a skilled artisan would understand these terms.

The term "adjacent," when used herein to refer two nucleotide sequences in a nucleic acid, can refer to nucleotide sequences separated by 0 to about 20 nucleotides, more specifically, in a range of about 1 to about 10 nucleotides, or sequences that directly abut one another.

The term "nucleic acid" refers to a nucleotide polymer, and unless otherwise limited, includes known analogs of natural nucleotides that can function in a similar manner (e.g., hybridize) to naturally occurring nucleotides.

The term nucleic acid includes any form of DNA or RNA, including, for example, genomic DNA; complementary DNA (cDNA), which is a DNA representation of mRNA, usually obtained by reverse transcription of messenger RNA (mRNA) or by amplification; DNA molecules produced synthetically or by amplification; and mRNA.

The term nucleic acid encompasses double- or triple-stranded nucleic acids, as well as single-stranded molecules. In double- or triple-stranded nucleic acids, the nucleic acid strands need not be coextensive (i.e, a double-stranded nucleic acid need not be double-stranded along the entire length of both strands).

The term nucleic acid also encompasses any chemical modification thereof, such as by methylation and/or by capping. Nucleic acid modifications can include addition of chemical groups that incorporate additional charge, polarizability, hydrogen bonding, electrostatic interaction, and functionality to the individual nucleic acid bases or to the nucleic acid as a whole. Such modifications may include base modifications such as 2'-position sugar modifications, 5-position pyrimidine modifications, 8-position purine modifications, modifications at cytosine exocyclic amines, substitutions of 5-bromo-uracil, backbone modifications, unusual base pairing combinations such as the isobases isocytidine and isoguanidine, and the like.

More particularly, in certain embodiments, nucleic acids, can include polydeoxyribonucleotides (containing 2-deoxy-D-ribose), polyribonucleotides (containing D-ribose), and any other type of nucleic acid that is an N- or C-glycoside of a purine or pyrimidine base, as well as other polymers containing nonnucleotidic backbones, for example, polyamide (e.g., peptide nucleic acids (PNAs)) and polymorpholino (commercially available from the Anti-Virals, Inc., Corvallis, Oregon, as Neugene) polymers, and other synthetic sequence-specific nucleic acid polymers providing that the polymers contain nucleobases in a configuration which allows for base pairing and base stacking, such as is found in DNA and RNA. The term nucleic acid also encompasses linked nucleic acids (LNAs), which are described in U.S. Patent Nos. 6,794,499, 6,670,461, 6,262,490, and 6,770,748.

The nucleic acid(s) can be derived from a completely chemical synthesis process, such as a solid phase-mediated chemical synthesis, from a biological source, such as through isolation from any species that produces nucleic acid, or from processes that involve the manipulation of nucleic acids by molecular biology tools, such as DNA replication, PCR amplification, reverse transcription, or from a combination of those processes.

The term "sample nucleic acids" can to refer to nucleic acids (1) in a sample taken directly from a subject, (2) in a fraction of a sample taken directly from a subject, and (3) in a sample, or fraction thereof, that has been subjected to a treatment, such as, e.g., preamplification. Where it is necessary to distinguish among these meanings, clarifying language is used; for example, a "preamplified" sample" or "preamplified" nucleic acids refer to a sample or nucleic acids that have been subjected to preamplification.

The term "target nucleic acids" is used herein to refer to particular nucleic acids to be detected in the methods described herein.

As used herein the term "target nucleotide sequence" refers to a molecule that includes the nucleotide sequence of a target nucleic acid, such as, for example, the amplification product obtained by amplifying a target nucleic acid or the cDNA produced upon reverse transcription of an RNA target nucleic acid.

As used herein, the term "complementary" refers to the capacity for precise pairing between two nucleotides. I.e., if a nucleotide at a given position of a nucleic acid is capable of hydrogen bonding with a nucleotide of another nucleic acid, then the two nucleic acids are considered to be complementary to one another at that position. Complementarity between two single-stranded nucleic acid molecules may be "partial," in which only some of the nucleotides bind, or it may be complete when total complementarity exists between the single-stranded molecules. The degree of complementarity between nucleic acid strands has significant effects on the efficiency and strength of hybridization between nucleic acid strands.

"Specific hybridization" refers to the binding of a nucleic acid to a target nucleotide sequence in the absence of substantial binding to other nucleotide sequences present in the hybridization mixture under defined stringency conditions. Those of skill in the art recognize that relaxing the stringency of the hybridization conditions allows sequence mismatches to be tolerated.

In particular embodiments, hybridizations are carried out under stringent hybridization conditions. The phrase "stringent hybridization conditions" generally refers to a temperature in a range from about 5°C to about 20°C or 25°C below than the melting temperature (Tₘ) for a specific sequence at a defined ionic strength and pH. As used herein, the Tₘ is the temperature at which a population of double-stranded nucleic acid molecules becomes half-dissociated into single strands. Methods for calculating the Tₘ of nucleic acids are well known in the art (see, e.g., Berger and Kimmel (1987) METHODS IN ENZYMOLOGY, VOL.152: GUIDE TO MOLECULAR CLONING TECHNIQUES, San Diego: Academic Press, Inc. and Sambrook et al. (1989) MOLECULAR CLONING: A LABORATORY MANUAL, 2ND ED., VOLS. 1-3, Cold Spring Harbor Laboratory). As indicated by standard references, a simple estimate of the Tₘ value may be calculated by the equation: Tₘ=81.5+0.41(% G+C), when a nucleic acid is in aqueous solution at 1 M NaCl (see, e.g., Anderson and Young, Quantitative Filter Hybridization in NUCLEIC ACID HYBRIDIZATION (1985)). The melting temperature of a hybrid (and thus the conditions for stringent hybridization) is affected by various factors such as the length and nature (DNA, RNA, base composition) of the primer or probe and nature of the target nucleic acid (DNA, RNA, base composition, present in solution or immobilized, and the like), as well as the concentration of salts and other components (e.g., the presence or absence of formamide, dextran sulfate, polyethylene glycol). The effects of these factors are well known and are discussed in standard references in the art. Illustrative stringent conditions suitable for achieving specific hybridization of most sequences are: a temperature of at least about 60°C and a salt concentration of about 0.2 molar at pH7.

Non-coding RNAs include those RNA species that are not necessarily translated into protein. These include, but are not limited to, transfer RNA (tRNA) and ribosomal RNA (rRNA), as well as RNAs such as small nucleolar RNAs (snoRNA; e.g., those associated with methylation or pseudouridylation), microRNAs (miRNA; which regulate gene expression), small interfering RNAs (siRNAs; which are involved in the RNA interference (RNAi) pathway, where they interfere with the expression of specific genes, but have also been shown to act as antiviral agents and in shaping the chromatin structure of a genome) and Piwi-interacting RNAs (piRNAs; which form RNA-protein complexes through interactions with Piwi proteins; these piRNA complexes have been linked to transcriptional gene silencing of retrotransposons and other genetic elements in germ line cells, particularly those in spermatogenesis), and long non-coding RNAs (long ncRNAs; which are non-coding transcripts that are typically longer than about 200 nucleotides).

The term "oligonucleotide" is used to refer to a nucleic acid that is relatively short, generally shorter than 200 nucleotides, more particularly, shorter than 100 nucleotides, most particularly, shorter than 50 nucleotides. Typically, oligonucleotides are single-stranded DNA molecules.

The term "primer" refers to an oligonucleotide that is capable of hybridizing (also termed "annealing") with a nucleic acid and serving as an initiation site for nucleotide (RNA or DNA) polymerization under appropriate conditions (i.e., in the presence of four different nucleoside triphosphates and an agent for polymerization, such as DNA or RNA polymerase or reverse transcriptase) in an appropriate buffer and at a suitable temperature. The appropriate length of a primer depends on the intended use of the primer, but primers are typically at least 7 nucleotides long and, more typically range from 10 to 30 nucleotides, or even more typically from 15 to 30 nucleotides, in length. Other primers can be somewhat longer, e.g., 30 to 50 nucleotides long. In this context, "primer length" refers to the portion of an oligonucleotide or nucleic acid that hybridizes to a complementary "target" sequence and primes nucleotide synthesis. Short primer molecules generally require cooler temperatures to form sufficiently stable hybrid complexes with the template. A primer need not reflect the exact sequence of the template but must be sufficiently complementary to hybridize with a template. The term "primer site" or "primer binding site" refers to the segment of the target nucleic acid to which a primer hybridizes.

A primer is said to anneal to another nucleic acid if the primer, or a portion thereof, hybridizes to a nucleotide sequence within the nucleic acid. The statement that a primer hybridizes to a particular nucleotide sequence is not intended to imply that the primer hybridizes either completely or exclusively to that nucleotide sequence. For example, in certain embodiments, amplification primers used herein are said to "anneal to a nucleotide tag." This description encompasses primers that anneal wholly to the nucleotide tag, as well as primers that anneal partially to the nucleotide tag and partially to an adjacent nucleotide sequence, e.g., a target nucleotide sequence. Such hybrid primers can increase the specificity of the amplification reaction.

The term "primer pair" refers to a set of primers including a 5' "upstream primer" or "forward primer" that hybridizes with the complement of the 5' end of the DNA sequence to be amplified and a 3' "downstream primer" or "reverse primer" that hybridizes with the 3' end of the sequence to be amplified. As will be recognized by those of skill in the art, the terms "upstream" and "downstream" or "forward" and "reverse" are not intended to be limiting, but rather provide illustrative orientation in particular embodiments.

A "probe" is a nucleic acid capable of binding to a target nucleic acid of complementary sequence through one or more types of chemical bonds, generally through complementary base pairing, usually through hydrogen bond formation, thus forming a duplex structure. The probe binds or hybridizes to a "probe binding site." The probe can be labeled with a detectable label to permit facile detection of the probe, particularly once the probe has hybridized to its complementary target. Alternatively, however, the probe may be unlabeled, but may be detectable by specific binding with a ligand that is labeled, either directly or indirectly. Probes can vary significantly in size. Generally, probes are at least 7 to 15 nucleotides in length. Other probes are at least 20, 30, or 40 nucleotides long. Still other probes are somewhat longer, being at least 50, 60, 70, 80, or 90 nucleotides long. Yet other probes are longer still, and are at least 100, 150, 200 or more nucleotides long. Probes can also be of any length that is within any range bounded by any of the above values (e.g., 15-20 nucleotides in length).

The primer or probe can be perfectly complementary to the target nucleic acid sequence or can be less than perfectly complementary. In certain embodiments, the primer has at least 65% identity to the complement of the target nucleic acid sequence over a sequence of at least 7 nucleotides, more typically over a sequence in the range of 10-30 nucleotides, and often over a sequence of at least 14-25 nucleotides, and more often has at least 75% identity, at least 85% identity, at least 90% identity, or at least 95%, 96%, 97%. 98%, or 99% identity. It will be understood that certain bases (e.g., the 3' base of a primer) are generally desirably perfectly complementary to corresponding bases of the target nucleic acid sequence. Primer and probes typically anneal to the target sequence under stringent hybridization conditions.

The term "nucleotide tag" is used herein to refer to a predetermined nucleotide sequence that is added to a target nucleotide sequence. The nucleotide tag can encode an item of information about the target nucleotide sequence, such the identity of the target nucleotide sequence or the identity of the sample from which the target nucleotide sequence was derived. In certain embodiments, such information may be encoded in one or more nucleotide tags, e.g., a combination of two nucleotide tags, one on either end of a target nucleotide sequence, can encode the identity of the target nucleotide sequence.

As used herein, the term "encoding reaction" refers to reaction in which at least one nucleotide tag is added to a target nucleotide sequence. Nucleotide tags can be added, for example, by an "encoding PCR" in which the at least one primer comprises a target-specific portion and a nucleotide tag located on the 5' end of the target-specific portion, and a second primer that comprises only a target-specific portion or a target-specific portion and a nucleotide tag located on the 5' end of the target-specific portion. For illustrative examples of PCR protocols applicable to encoding PCR, see pending WO Application US03/37808 as well as U.S. Pat. No.6,605,451. Nucleotide tags can also be added by an "encoding ligation" reaction that can comprise a ligation reaction in which at least one primer comprises a target-specific portion and nucleotide tag located on the 5' end of the target-specific portion, and a second primer that comprises a target-specific portion only or a target-specific portion and a nucleotide tag located on the 5' end of the target specific portion. Illustrative encoding ligation reactions are described, for example, in U.S. Patent Publication No. 2005/0260640.

As used herein an "encoding reaction" produces a "tagged target nucleotide sequence," which includes a nucleotide tag linked to a target nucleotide sequence.

As used herein the term "barcode" refers to a specific nucleotide sequence that encodes information about an amplicon produce during preamplification or amplification. To introduce a barcode into an amplicon, "barcode primer" that includes the barcode nucleotide sequence can be employed in an amplification reaction. For example, a different barcode primer can be employed to amplify one or more target sequences from each of a number of different samples, such that the barcode nucleotide sequence indicates the sample origin of the resulting amplicons.

The term "melting temperature discriminator sequence" refers to a subsequence of a longer double-stranded polynucleotide that renders that polynucleotide distinguishable, by melting temperature, from another polynucleotide, e.g. one containing a different melting temperature discriminator sequence.

As used herein with reference to a portion of a primer, the term "target-specific" nucleotide sequence refers to a sequence that can specifically anneal to a target nucleic acid or a target nucleotide sequence under suitable annealing conditions.

As used herein with reference to a portion of a primer, the term "nucleotide tag-specific nucleotide sequence" refers to a sequence that can specifically anneal to a nucleotide tag under suitable annealing conditions.

Amplification according to the present teachings encompasses any means by which at least a part of at least one target nucleic acid is reproduced, typically in a template-dependent manner, including without limitation, a broad range of techniques for amplifying nucleic acid sequences, either linearly or exponentially. Illustrative means for performing an amplifying step include ligase chain reaction (LCR), ligase detection reaction (LDR), ligation followed by Q-replicase amplification, PCR, primer extension, strand displacement amplification (SDA), hyperbranched strand displacement amplification, multiple displacement amplification (MDA), nucleic acid strand-based amplification (NASBA), two-step multiplexed amplifications, rolling circle amplification (RCA), and the like, including multiplex versions and combinations thereof, for example but not limited to, OLA/PCR, PCR/OLA, LDR/PCR, PCR/PCR/LDR, PCR/LDR, LCR/PCR, PCR/LCR (also known as combined chain reaction--CCR), and the like. Descriptions of such techniques can be found in, among other sources, Ausbel et al.; PCR Primer: A Laboratory Manual, Diffenbach, Ed., Cold Spring Harbor Press (1995); The Electronic Protocol Book, Chang Bioscience (2002); Msuih et al., J. Clin. Micro. 34:501-07 (1996); The Nucleic Acid Protocols Handbook, R. Rapley, ed., Humana Press, Totowa, N.J. (2002); Abramson et al., Curr Opin Biotechnol. 1993 Feb.;4(1):41-7, U.S. Pat. No. 6,027,998; U.S. Pat. No. 6,605,451, Barany et al., PCT Publication No. WO 97/31256; Wenz et al., PCT Publication No. WO 01/92579; Day et al., Genomics, 29(1): 152-162 (1995), Ehrlich et al., Science 252:1643-50 (1991); Innis et al., PCR Protocols: A Guide to Methods and Applications, Academic Press (1990); Favis et al., Nature Biotechnology 18:561-64 (2000); and Rabenau et al., Infection 28:97-102 (2000); Belgrader, Barany, and Lubin, Development of a Multiplex Ligation Detection Reaction DNA Typing Assay, Sixth International Symposium on Human Identification, 1995 (available on the world wide web at: promega.com/geneticidproc/ussymp6proc/blegrad.html- ); LCR Kit Instruction Manual, Cat. #200520, Rev. #050002, Stratagene, 2002; Barany, Proc. Natl. Acad. Sci. USA 88:188-93 (1991); Bi and Sambrook, Nucl. Acids Res. 25:2924-2951 (1997); Zirvi et al., Nucl. Acid Res. 27:e40i-viii (1999); Dean et al., Proc Natl Acad Sci USA 99:5261-66 (2002); Barany and Gelfand, Gene 109:1-11 (1991); Walker et al., Nucl. Acid Res. 20:1691-96 (1992); Polstra et al., BMC Inf. Dis. 2:18- (2002); Lage et al., Genome Res. 2003 Feb.;13(2):294-307, and Landegren et al., Science 241:1077-80 (1988), Demidov, V., Expert Rev Mol Diagn. 2002 Nov.;2(6):542-8., Cook et al., J Microbiol Methods. 2003 May;53(2):165-74, Schweitzer et al., Curr Opin Biotechnol. 2001 Feb.;12(1):21-7, U.S. Pat. No. 5,830,711, U.S. Pat. No. 6,027,889, U.S. Pat. No. 5,686,243, PCT Publication No. WO0056927A3, and PCT Publication No. WO9803673A1.

In some embodiments, amplification comprises at least one cycle of the sequential procedures of: annealing at least one primer with complementary or substantially complementary sequences in at least one target nucleic acid; synthesizing at least one strand of nucleotides in a template-dependent manner using a polymerase; and denaturing the newly-formed nucleic acid duplex to separate the strands. The cycle may or may not be repeated. Amplification can comprise thermocycling or can be performed isothermally.

The term "qPCR" is used herein to refer to quantitative real-time polymerase chain reaction (PCR), which is also known as "real-time PCR" or "kinetic polymerase chain reaction."

A "reagent" refers broadly to any agent used in a reaction, other than the analyte (e.g., nucleic acid being analyzed). Illustrative reagents for a nucleic acid amplification reaction include, but are not limited to, buffer, metal ions, polymerase, reverse transcriptase, primers, template nucleic acid, nucleotides, labels, dyes, nucleases, and the like. Reagents for enzyme reactions include, for example, substrates, cofactors, buffer, metal ions, inhibitors, and activators.

The term "universal detection probe" is used herein to refer to any probe that identifies the presence of an amplification product, regardless of the identity of the target nucleotide sequence present in the product.

The term "universal qPCR probe" is used herein to refer to any such probe that identifies the presence of an amplification product during qPCR. In particular embodiments, nucleotide tags according to the invention can include a nucleotide sequence to which a detection probe, such as a universal qPCR probe binds. Where a tag is added to both ends of a target nucleotide sequence, each tag can, if desired, include a sequence recognized by a detection probe. The combination of such sequences can encode information about the identity or sample source of the tagged target nucleotide sequence. In other embodiments, one or more amplification primers can include a nucleotide sequence to which a detection probe, such as a universal qPCR probe binds. In this manner, one, two, or more probe binding sites can be added to an amplification product during the amplification step of the methods of the invention. Those of skill in the art recognize that the possibility of introducing multiple probe binding sites during preamplification (if carried out) and amplification facilitates multiplex detection, wherein two or more different amplification products can be detected in a given amplification mixture or aliquot thereof.

The term "universal detection probe" is also intended to encompass primers labeled with a detectable label (e.g., a fluorescent label), as well as non-sequence-specific probes, such as DNA binding dyes, including double-stranded DNA (dsDNA) dyes, such as SYBR Green.

The term "target-specific qPCR probe" is used herein to refer to a qPCR probe that identifies the presence of an amplification product during qPCR, based on hybridization of the qPCR probe to a target nucleotide sequence present in the product.

"Hydrolysis probes" are generally described in U.S. Patent No. 5,210,015. Hydrolysis probes take advantage of the 5'-nuclease activity present in the thermostable Taq polymerase enzyme typically used in the PCR reaction (TaqMan^{®} probe technology, Applied Biosystems, Foster City CA). The hydrolysis probe is labeled with a fluorescent detector dye such as fluorescin, and an acceptor dye or quencher. In general, the fluorescent dye is covalently attached to the 5' end of the probe and the quencher is attached to the 3' end of the probe, and when the probe is intact, the fluorescence of the detector dye is quenched by fluorescence resonance energy transfer (FRET). The probe anneals downstream of one of the primers that defines one end of the target nucleic acid in a PCR reaction. Using the polymerase activity of the Taq enzyme, amplification of the target nucleic acid is directed by one primer that is upstream of the probe and a second primer that is downstream of the probe but anneals to the opposite strand of the target nucleic acid. As the upstream primer is extended, the Taq polymerase reaches the region where the labeled probe is annealed, recognizes the probe-template hybrid as a substrate, and hydrolyzes phosphodiester bonds of the probe. The hydrolysis reaction irrevocably releases the quenching effect of the quencher dye on the reporter dye, thus resulting in increasing detector fluorescence with each successive PCR cycle. In particular, hydrolysis probes suitable for use in the invention can be capable of detecting 8-mer or 9-mer motifs that are common in the human and other genomes and/or transcriptomes and can have a high Tₘ of about 70°C enabled by the use of linked nucleic acid (LNA) analogs.

The term "label," as used herein, refers to any atom or molecule that can be used to provide a detectable and/or quantifiable signal. In particular, the label can be attached, directly or indirectly, to a nucleic acid or protein. Suitable labels that can be attached to probes include, but are not limited to, radioisotopes, fluorophores, chromophores, mass labels, electron dense particles, magnetic particles, spin labels, molecules that emit chemiluminescence, electrochemically active molecules, enzymes, cofactors, and enzyme substrates.

The term "dye," as used herein, generally refers to any organic or inorganic molecule that absorbs electromagnetic radiation at a wavelength greater than or equal 340 nm.

The term "fluorescent dye," as used herein, generally refers to any dye that emits electromagnetic radiation of longer wavelength by a fluorescent mechanism upon irradiation by a source of electromagnetic radiation, such as a lamp, a photodiode, or a laser.

The term "elastomer" has the general meaning used in the art. Thus, for example, Allcock et al. (Contemporary Polymer Chemistry, 2nd Ed.) describes elastomers in general as polymers existing at a temperature between their glass transition temperature and liquefaction temperature. Elastomeric materials exhibit elastic properties because the polymer chains readily undergo torsional motion to permit uncoiling of the backbone chains in response to a force, with the backbone chains recoiling to assume the prior shape in the absence of the force. In general, elastomers deform when force is applied, but then return to their original shape when the force is removed.

A "polymorphic marker" or "polymorphic site" is a locus at which nucleotide sequence divergence occurs. Illustrative markers have at least two alleles, each occurring at frequency of greater than 1%, and more typically greater than 10% or 20% of a selected population. A polymorphic site may be as small as one base pair. Polymorphic markers include restriction fragment length polymorphism (RFLPs), variable number of tandem repeats (VNTR's), hypervariable regions, minisatellites, dinucleotide repeats, trinucleotide repeats, tetranucleotide repeats, simple sequence repeats, deletions, and insertion elements such as Alu. The first identified allelic form is arbitrarily designated as the reference form and other allelic forms are designated as alternative or variant alleles. The allelic form occurring most frequently in a selected population is sometimes referred to as the wildtype form. Diploid organisms may be homozygous or heterozygous for allelic forms. A diallelic polymorphism has two forms. A triallelic polymorphism has three forms.

A "single nucleotide polymorphism" (SNP) occurs at a polymorphic site occupied by a single nucleotide, which is the site of variation between allelic sequences. The site is usually preceded by and followed by highly conserved sequences of the allele (e.g., sequences that vary in less than 1/100 or 1/1000 members of the populations). A SNP usually arises due to substitution of one nucleotide for another at the polymorphic site. A transition is the replacement of one purine by another purine or one pyrimidine by another pyrimidine. A transversion is the replacement of a purine by a pyrimidine or vice versa. SNPs can also arise from a deletion of a nucleotide or an insertion of a nucleotide relative to a reference allele.

As used herein, the phrase "the relative copy numbers of the target nucleic acids is substantially maintained" and like phrases indicate that the copy numbers of the target nucleic acids, relative to one another are sufficiently maintained to permit reproducible copy number determinations for the target nucleic acids using the methods described herein.

The term "chromosome-specific motif" is used herein to refer to a nucleotide sequence that is used to identify the presence of a particular chromosome. The motif can, but need not, be absolutely chromosome-specific, such that the motif can be used to unambiguously identify the chromosome, regardless of the presence of other chromosome sequences in an assay mixture. Alternatively, the motif can be one that simply distinguishes one chromosome from another chromosome who sequences of are present in an assay mixture.

### General Strategies for Analyzing Scarce Target Nucleic Acids in a Sample

In certain embodiments, methods of the invention can include one or more of the following strategies for analyzing scarce target nucleic acids in a sample in combination with a step that selectively enriches for short (less than 300 nucleotides) nucleic acids. This selective enrichment step is as defined in the claims. In some embodiments, methods of the invention can entail the enrichment method of claim 1, alone or in combination with another enrichment method. Other detection and quantification methods that can be combined with the enrichment methods described herein are found in commonly owned, co-pending Application Nos. 12/548,132 (filed 8/26/2009; Attorney Docket No. FLUDP002), 12/687,018 (filed 1/13/2010; Attorney Docket No. FLUDP005), 12/695,010 (filed 1/27/2010; Attorney Docket No. FLUDP006), 12/753,703 (filed 4/2/2010; Attorney Docket No. FLUDP007), and 12/752,974 (filed 4/1/2010; Attorney Docket No. FLUDP008).

### General Approaches for Increasing the Accuracy and /or Precision of Relative Copy Number Determination by Amplification

The detection of fetal aneuploidy in a maternal bodily fluid sample (e.g., plasma) requires a significantly higher assay accuracy and precision than has been achieved previously. The methods described herein facilitate the detection of copy number differences of less than 1.5-fold. In various embodiments, the methods permit detection of copy number differences of 1.45-fold, 1.4-fold, 1.35-fold, 1.3-fold, 1.25-fold, 1.2-fold, 1.15-fold, 1.1-fold, 1.09-fold, 1.08-fold, 1.07-fold, 1.06-fold, 1.05-fold, 1.04-fold, 1.03-fold, or 1.02-fold or less, or a copy number difference falling within any range bounded by any two of the above values. The required precision is readily achieved using one or more of the several approaches described herein, individually or in combination.

First, one can preamplify the target nucleic acid sequence before analysis by amplification. Preamplification increases the number of target and/or internal control nucleic acids, which renders subsequent relative copy number determinations more accurate and precise. In particular embodiments, the target sequence and an internal control sequence are preamplified in parallel, typically, at the same time, under the same reaction conditions, and, more typically, in the same reaction mixture. Generally, the preamplification is carried out for a relatively small number of cycles, so that the relative amounts of the target and internal control sequences is substantially unaltered by the preamplification step. More specifically, the preamplification should be sufficiently proportionate that copy number differences of less than 1.5-fold can be detected in the subsequent amplification reaction. In various embodiments, preamplification is carried out for between 5 and 25 cycles, e.g., for 5, 6, 7, 8, 9, 10, 11, 12, 13, 14, 15, 16, 17, 18, 19, 20, 21, 22, 23, 24, or 25 cycles. In illustrative embodiments, preamplification is carried out for between 10 and 20 cycles.

A second approach to increase the accuracy and/or precision of the relative copy number determination is to carry out a large number of parallel preamplification and/or amplification reactions (i.e., replicates). The use of replicates in preamplification can increase the accuracy of the subsequent relative copy number determination, and the use or replicates during amplification/quantification can increase the precision of this determination. In specific embodiments, each preamplification and/or amplification reaction (i.e., for each sample and/or each nucleic acid sequence of interest) is carried out in at least 4, 6, 8, 10, 12, 16, 24, 32, 48, 50, 100, 200, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, or 10,000 or more replicates. Furthermore, the number of replicates can be within any range having any of these values as endpoints.

In illustrative embodiments, a sample is divided into aliquots and preamplified, and then each preamplified aliquot is divided into further aliquots and subjected to amplification.

An approach to increasing the accuracy and precision of aneuploidy determinations is to analyze a plurality of target sequences on the chromosome of interest. In illustrative embodiments, 2, 3, 4, 5, 6, 7, 8, 9, 10, 20, 30, 40, 50, 60, 70, 80, 90, 100, 125, 150, 175, 200, 225, 250, 275, 300, 325, 350, 375, 400, 425, 450, 475, 500, 550, 600, 650, 700, 750, 800, 850, 900, 950, 1000 or more target and/or internal control sequences on a chromosome of interest are analyzed. In addition, any number of sequences falling within ranges bounded by any of these values can be analyzed.

### Considerations for Preamplification/Amplification

In certain embodiments, the length of the target and/or internal control sequences is relatively short, e.g., such that preamplification and/or amplification produces amplicons including fewer than 200, 175, 150, 125, 100, 75, 50, 45, 40, 35, or 30 nucleotides or amplicons having a length within any range bounded by these values. In specific embodiments, primer pairs wherein the primers bind to overlapping target sequences can be employed. The overlap can be, e.g., 1, 2, or 3 nucleotides. Assay methods employing small amplicons are useful for applications aimed at determining copy number in samples containing fragmented nucleic acids, as is the case, e.g., for cell-free fetal DNA in a maternal bodily fluid (e.g., plasma), cell-free DNA in the bodily fluid (e.g., plasma) of subjects with cancer, or DNA from formalin-fixed paraffin-embedded tissue.

Relatively long annealing times and/or lower than usual annealing temperatures can be employed in particular embodiments, e.g., where the target and/or internal control sequences are present at a relatively low concentration in the sample (e.g., as in the case of cell-free fetal DNA in maternal plasma). In illustrative embodiments, these conditions can be employed, individually or together, during preamplification. Illustrative longer-than-usual annealing times include more than 30 seconds, and more than 60 seconds, more than 120 seconds, more than 240 seconds, more than 10 minutes, more than 1 hour, or more than 10 hours, or any time falling within a range bounded by any of these values. Longer annealing times are typically employed in highly multiplexed reactions and/or reactions where primer concentrations are relatively low. Illustrative lower-than-usual annealing temperatures include less than 65°C, less than 60°C, less than 55°C, less than 50°C, and less than any temperature falling within a range bounded by any of these values.

In particular embodiments, the preamplification step can be used to introduce a nucleotide tag. For example, at least one primer of each primer pair employed for preamplification can include a nucleotide tag, which becomes incorporated into the preamplified nucleic acids. The nucleotide tag can include any desired sequence, e.g., one that encodes an item of information about the target and/or internal control sequence and/or one that includes a primer binding site and/or a probe binding site. In illustrative embodiments, the nucleotide tag includes a universal tag and/or a common tag. A common tag can be introduced into a plurality of target and/internal control sequences. For example, a common chromosome-specific tag can be introduced into all sequences preamplified from a particular chromosome.

To introduce one or more nucleotide tags during preamplification, one or more primers include a target-specific portion and a nucleotide tag. In the first cycle of amplification, only the target-specific portion anneals to the target nucleic acid sequence (or internal control sequence). If both primers in each primer pair are tagged, the same is true for the second cycle of amplification. During these cycles, the annealing temperature should be suitable for annealing of the target-specific portion(s) of the primer(s). Subsequently, however, the annealing temperature can be increased to increase the stringency of the annealing, and thereby favor the amplification of tagged target and/or tagged internal control sequences.

If one or more tags is/are introduced into each target and/or internal control sequence, amplification/quantification can be carried out using one or more tag-specific primers. So, for example, if common nucleotide tags are employed, common tag-specific primers can be used to produce amplicons for detection. Such primers could introduce a binding site for a universal detection probe such that detection could be carried out using a single probe for multiple sequences.

### Enhancing Target Sequence Populations in a Sample of Mixed Length Nucleic Acids

Methods are provided for enhancing a nucleic acid sample for target sequences of interest and/or selectively tagging those sequences. These enrichment/selective tagging methods can be combined with methods described above to further facilitate the detection and or quantification of target sequences in samples having mixed length nucleic acids (*e.g.* fetal DNA in maternal plasma or tumor DNA in plasma from cancer patients.

In certain embodiments, methods are provided for protecting target sequences from exonuclease digestion thereby facilitating the elimination in a sample of undesired amplification primers and/or a portion of certain background sequences (e.g., maternal DNA).

Methods are also provided for selectively tagging short *(e.g.,* fetal DNA) sequences in a sample comprising long and short nucleic acids by using inner tagged forward and reverse primers (one or both tagged) in combination with outer primers in a nucleic acid amplification *(e.g.,* PCR) mix. As explained below, shorter *(e.g.,* fetal) target nucleic acids are amplified and tagged while the amplification of longer (e.g., maternal nucleic acid sequences) is suppressed by one or more mechanisms including blocking of extension of the inner primers by prior annealing and extension of the outer primers, TaqMan 5' endonuclease digestion of the inner primer and/or its extension product by extension of the outer primer, and/or displacement of the inner tagged product and exonuclease digestion after amplification cycle 1 or 2.

Some embodiments, entail the use of one or more outer primers (or capture probe, i.e. no amplification need be carried out) linked to a moiety that can be used to remove these sequences (e.g., biotin). Alternatively, one or more inner primer may be linked to such a moiety. If such (an inner or outer) primer is extended prior to separation, it may or may not be separated from target sequence. Extension can be carried out to provide a stronger binding to the target sequence.

### Selective Protection of Target Sequences from Enzymatic Degradation

In certain embodiments methods are provided for the selective protection of target nucleic acid sequences from enzymatic degradation. Accordingly, in certain embodiments, the methods comprise denaturing sample nucleic acids in a reaction mixture; contacting the denatured sample nucleic acids with at least one target-specific primer pair under suitable annealing conditions; conducting a first cycle of extension of any annealed target-specific primer pairs by nucleotide polymerization; and after the first cycle of extension, conducting a first cycle of nuclease digestion of single-stranded nucleic acid sequences in the reaction mixture. In various embodiments the methods can further involve denaturing the nucleic acids in the reaction mixture after the first cycle of nuclease digestion; contacting the denatured nucleic acids with at least one target-specific primer pair under suitable annealing conditions; conducting a second cycle of extension of any annealed target-specific primer pairs by nucleotide polymerization; and conducting a second cycle of nuclease digestion of single-stranded nucleic acid sequences in the reaction mixture. The process can optionally be repeated for additional cycles as required. In certain embodiments the same target-specific primer pair is used to prime each of the first and second cycles of extension, while in other embodiments, different target-specific primer pairs are used for the first and second cycle. Any of a variety of nucleases that preferably digest single stranded nucleic acids can be used. Suitable nucleases include for example a single strand-specific 3' exonuclease, a single strand-specific endonuclease, a single strand-specific 5' exonuclease, and the like. In certain embodiments the nuclease comprises *E. coli* Exonuclease I. In certain embodiments the nuclease comprises a reagent such as ExoSAP-IT®. ExoSAP-IT® utilizes two hydrolytic enzymes, Exonuclease I and Shrimp Alkaline Phosphatase, together in a specially formulated buffer to remove unwanted dNTPs and primers from PCR products. Exonuclease I removes residual single-stranded primers and any extraneous single-stranded DNA produced in the PCR. Shrimp Alkaline Phosphatase removes the remaining dNTPs from the PCR mixture. In certain embodiments ExoSAP-IT is added directly to the PCR product and incubated at 37°C for 15 minutes. After PCR treatment, ExoSAP-IT® is inactivated simply by heating, *e.g.,* to 80°C for 15 minutes.

In certain embodiments the target-specific primers comprise dU, rather than dT, and dUTP, rather than dTTP, is present in the reaction mixture. In certain embodiments the methods additionally comprise contacting the reaction mixture with *E. coli* Uracil-N-Glycosylase after the second cycle of nuclease digestion. In one illustrative embodiment, the method is carried out using two or more target-specific primer pairs, where each primer pair is specific for a different target nucleotide sequence. In various embodiments, particular, where the target specific primers introduced nucleotide tags, the method can involve after the second cycle of nuclease digestion, denaturing the nucleic acids in the reaction mixture; contacting the denatured nucleic acids with at least one target *(e.g.,* tag) specific primer pair under suitable annealing conditions; and amplifying the corresponding *(e.g.,* tagged) target nucleotide sequence.

In certain embodiments, "primers" (or probes) that hybridize to target need not be extended. If, for example, 3'-exonuclease is employed, the primer will block digestion of the target strand at a certain position, which will become the 3' end of the remaining target strand, while all sequences upstream of the target will be protected, whether double stranded (paired with primer/probe) or single stranded.

### Selective Tagging of Short Target Sequences

In certain embodiments methods are provided for selectively tagging short target sequences (*e.g*., cell free fetal DNA) in a mixed population of short and long nucleic acids *(e.g.,* cell free DNA obtained from maternal plasma). In various embodiments the method typically involves performing a nucleic acid amplification using a set of nested primers comprising inner primers and outer primers. In various embodiments one or both of the inner can be tagged to thereby introduce a tag onto the target amplification product.

The outer primers do not anneal on the short fragments (*e.g*., fetal DNA) that carry the (inner) target sequence. The inner primers (labeled "I" in the figure) anneal to the short fragments and generate an amplification product that carries a tag and the target sequence. After 2 cycles a short double stranded fragment generates two double stranded products (which are 3'-exonuclease resistant). One strand of each of these carries both tags (where both primers were tagged).

At the same time, tagging of the long fragments (*e.g*., maternal DNA) is inhibited. This occurs through a combination of mechanisms. First, the extension of the inner primers can be blocked by the prior annealing and extension of the outer primer. Second, the extension of the outer primer can lead to cleavage of the tag from the already annealed inner primer. The third possibility is that the inner primers' extension product is displaced but intact. The result is that after two cycles, target sequences on the short nucleic acids *(e.g.,* cell free fetal DNA) are tagged, while the longer nucleic acids (*e.g.,* cell free maternal DNA), even those containing the target nucleotide sequence, are not tagged. Moreover, the tagged amplification products from the short sequences are double stranded and thereby 3'-exonuclease resistant.

At this point, enrichment for tagged target sequences (*e.g*., fetal DNA) can readily be accomplished by any of a variety of methods. For example, an exonuclease digestion can be performed (*e.g*., as described above) to digest all non-double stranded sequences including extension products of displaces inner primers. This removes the majority of genomic DNA background, while the target sequence are double stranded and stay intact. This also removes substantially all leftover primers.

In certain embodiments after the first cycle, and preferably after second cycle it is possible to directly continue thermocycling (*e.g*., without exonuclease digestion), but increasing the annealing temperature (*e.g*., from 60°C to 72°C). As a consequence, the inner primers will amplify only sequences that are tagged. The primers cannot bind to untagged target sequences.

In certain embodiments the denaturation temperature is selected to avoid melting of the long DNA amplification product(s). This can be applied right at the first cycle or after a limited amount of amplification rounds, when the short fragments have formed a PCR product that will melt at low temperatures (e.g., 70°C-80°C).

In certain embodiments the primers used for further amplification (e.g., after the first cycle and preferably after the second cycle) are specific to the two tags and not to the target sequences.

The resulting amplified tagged target sequences can be analyzed by any convenient methods. Such methods include, for example several modes of PCR (or other amplification methods). Several choices of how to encode target sequences by tagging can be selected. Straightforward is digital PCR. To multiplex several targets (*e.g.* per chromosome 21), these targets can be encoded with the same two tags. For each chromosome one could use only one primer pair in the PCR reaction.

Accordingly, in certain embodiments, methods are provided for selective tagging of short nucleic acids comprising a short target nucleotide sequence (nucleic acid) over longer nucleic acids comprising the same target nucleotide sequence. In various embodiments the method involves denaturing sample nucleic acids in a reaction mixture, where the sample nucleic acids comprise long nucleic acids and short nucleic acids, each comprising the same target nucleotide sequence. The denatured sample nucleic acids are contacted with one or preferably at least two target-specific primer pairs under suitable annealing conditions, where the primer pairs comprise an inner primer pair (one or both carrying a nucleotide tag, *e.g.,* a 5' nucleotide tag) that can amplify the target nucleotide sequence on long and short nucleic acids; and an outer primer pair that amplifies the target nucleotide sequence on long nucleic acids, but not on short nucleic acids. A first cycle of extension is conducted for any annealed primer pairs by nucleotide polymerization. After the first cycle of extension, the nucleic acids in the reaction mixture are denatured, the reaction mixture is subjected to suitable annealing conditions; and a second cycle of extension is conducted to produce at least one tagged target nucleotide sequence that comprises two nucleotide tags, one from each inner primer, with the target nucleotide sequence located between the nucleotide tags. It will be recognized that in certain embodiments, one use primers for only one strand in a simple mode, or for one strand per cycle.)

In certain embodiments, the method can additionally involve digesting single-stranded nucleic acid sequences in the reaction mixture after the first and/or the second cycle. In certain embodiments the digestion can by the use of an endonuclease (*e.g*., single strand-specific 3' exonuclease, single strand-specific endonuclease, a single strand-specific 5' exonuclease, a combination of exonuclease alkaline phosphatase, *etc*.), *e.g.,* as described above. The nuclease treatment digests substantially all non-double stranded sequences (including remaining primers, extension products of displaced inner primers, *etc.*), removes a substantial portion of gDNA background while leaving intact the double stranded target sequences.

In certain embodiments, as a substitute for the digestion, or in addition to the digestion, the method additionally comprises adding additional quantities the same or different target-specific primer pairs to the reaction mixture and performing one or more amplification cycles to preferentially amplify the tagged target sequences.

In certain embodiments after the first cycle of extension, any subsequent denaturation is carried out at a sufficiently low temperature (*e.g*. about 80°C to about 85°C) to avoid denaturation of any extension product of the outer primer pair.

In certain preferred embodiments, the method additionally comprises subjecting the reaction mixture to one or more cycles of amplification, wherein annealing is carried out at a sufficiently high temperature that the inner primers will only anneal to tagged target nucleotide sequences. This can be during the first to cycles and/or after the first two amplification cycles.

In certain embodiments the method(s) additionally involve contacting the at least one tagged target nucleotide sequence with a tag-specific primer pair under suitable annealing conditions; and amplifying the tagged target nucleotide sequence or using other modes of detection and/or quantification, *e.g*. as described herein. In certain embodiments the method further involves detecting and/or quantifying the amount of at least one tagged target nucleotide sequence produced by amplification (*e.g.*, via digital PCR (dPCR)).

The "short" nucleic acid fragments are about 300 nucleotides or shorter (e.g., 250 nt, 200 nt, *etc*.).

While the methods described herein can be used with essentially any nucleic acid sample comprising long and short nucleic acids (nucleic acid molecules), in certain embodiments, the short nucleic acids comprise fetal nucleic acids *(e.g.,* cell free fetal DNA from maternal plasma or urine), while the long nucleic acids comprise maternal nucleic acids *(e.g.,* cell free maternal DNA from plasma or urine). In various embodiments the nucleic acid are derived from a maternal biological sample *(e.g.,* a biological sample from a pregnant mammal (*e.g*., human) comprising maternal plasma, maternal urine, amniotic fluid, *etc.*)*.* In certain embodiments the nucleic acids are derived from a biological sample from a mammal (*e.g.*, a human or non-human mammal) having, suspected of having, or at risk for, a pathology or congenital disorder characterized by a nucleic acid abnormality (*e.g*., aneuploidy, fragmentation, amplification, deletion, single-nucleotide polymorphism, translocation, chromosomal rearrangement or resorting, *etc*.). In certain embodiments the nucleic acids are derived from a biological sample from a mammal (*e.g*., a human or non-human mammal) having, suspected of having, or at risk for a cancer. In certain embodiments, the short nucleic acid fragments comprise tumor or metastatic cell DNA, and the long nucleic acids comprise normal DNA.

In certain embodiments the method can be used to determine linkage of two sequence that are relatively neighboring. For example, if an upstream SNP has, for example a "G" nucleotide and the suppression primer(s) are designed to bind to this sequence then amplification of this SNP is suppressed. If the base is an A, the primers bind inefficiently and don't suppress indicating the presence of the A form sequence.

In various embodiments the inner and outer primers are designed/selected so the distance from outer primers to the target nucleotide sequence (measured as the number of nucleotides between the 5' ends and thereby including the length of both primers) ranges from about 50, 80, 100, 120, 130, 140, or 150 nucleotides or greater. In certain embodiments, the distance from outer primers to the target nucleotide ranges from about 50, 80, 100, 120, 130, 140, or 150 nucleotides to about 400, 350, 300, 250, or 200 nuclides. For selectively tagging fetal versus maternal cell free nucleic acids, the distance from each outer primer to the target nucleotide sequence is greater than about 130 nucleotides, and typically ranges from about 150 to about 200 nucleotides.

It will be recognized that, in certain embodiments, a large number of different target sequences (*e.g*., 2 or more, 3 or more, 5 or more, 10 or more, 15 or more, 20 or more, 50 or more, 100 or more per chromosome or other template(s)), can be tagged. Moreover using various tagging strategies, different amplification produces are readily discriminated thereby permitting the methods to be highly multiplexed.

In certain embodiments, fetal aneuploidy via Cts can be determined using for example tag-specific primers for pre-amplification (*e.g.* one primer pair for preamp after 2 tagging cycles), and then again using target specific primers for real-time PCR, *e.g.,* in a chip.

In certain embodiments it is contemplated to apply digital PCR (dPCR) or amplification and dPCR or fetal aneuploidy via CTS to the tagged short fragments. In certain illustrative embodiment the methods are not only useful for determining/detecting fetal aneuploidy but also for fetal genotyping (SNPs), mutation detection (including sequencing), methylation analysis, and the like.

In certain embodiments, inner primer can also be modified in another way, such that after 1, 2, 3, or more amplification cycles, products can be selectively removed from long targets. For example, an inner primer can be 5'-protected and long products digested by exonucleases. Alternatively, an inner prime can be modified (e.g., biotinylated) for capture.

Outer primers can be tagged such that they will not further amplify under the reaction conditions. For example, outer primers can be tagged with GC rich tags, so that the melting temperature (Tm) is above the T(denaturation) employed. Alternatively, outer primes can be designed such that the reverse complement product loops back onto itself, thereby being further extended by polymerase and forming a long stem that is not denatured or that closes again, thereby preventing annealing of inner primer and further amplification.

It is also possible to selectively tag the long sequences to remove them , including after a number of amplification cycles.

### Other Methods of Enriching for Short Nucleic Acids

### PEG Precipitation

One method of enriching for short nucleic acids is to subject a biological sample to polyethylene glycol (PEG) precipitation, as described, e.g., in Example 1 below. The method entails contacting a biological sample, fractionated or not, with approximately PEG in the presence of one or more monovalent salts under conditions sufficient to substantially precipitate large nucleic acids without substantially precipitating small (less than 300 nucleotides) nucleic acids. In various embodiments, PEG 8000 (MW 7000 - 9000) can be employed at a percentage ranging from about 1 to about 10 percent, e.g., about: 2, 3, 4, 5, 6, 7, 8, or 9 percent, or at a percentage falling within any range bounded by these values (e.g., 1-5%, 1-8%, etc.). A suitable salt concentration is, in specific embodiments, about 1 M monovalent salt (e.g., NaCl).

In certain embodiments, after addition of PEG, the mixture can be incubated at 4 °C / on ice (at -20 °C) for about 1 hour or longer (e.g., about 2, 5, 7, 10, 12 hours or overnight or for any duration falling within any range bounded by these values).

For greater separation of long and short nucleic acid fragments, the mixture can be centrifuged, e.g., at approx 1500 g (or higher) for 1-60 minutes. Suitable settings for centrifugation may vary depending on the particular application and can readily be determined by one of skill in the art. Short nucleic acids are in the supernatant. Larger molecular weight species are localized to the pellet. The length cutoff depends on the conditions, namely PEG 8000 concentration, which can be determined empirically, depending on the particular application. The supernatant can be removed to recover the short nucleic acids. If desired, short nucleic acids are further extracted and/or purified by standard methods prior to further manipulation and/or analysis (e.g., detection and/or quantification of nucleic acids.

### Nested Preamplification

A second enrichment method entails carrying out a first round of preamplification, e.g., as described above (which can include multiple cycles of preamplification), followed by a second round of preamplification (which can also include multiple cycles of preamplification). The second round of preamplification is, in certain embodiments, semi-nested (at least one primer in the second preamplification is inside the primers employed for the first one) or nested (both primers in the second preamplification is inside the primers employed for the first one) relative to the first round. The primers employed for these two rounds of preamplification contain one or more nucleotide tags, such that the two rounds of amplification produce tagged target amplicons. Thus, for example, a plurality of target nucleic acids can be preamplified, e.g., from the Down Syndrome critical region (DSCR) on chromosome and/or from chromosome 18, such that tags identify the resultant target amplicons as including DSCR or chromosome 18 sequences.

For example, using a 50-nucleotide target sequence, a first round of preamplification can be performed with tag on the forward primer (or optionally on the reverse primer, if it is desirable to increase annealing temperature) for 1 to 25 cycles. An optional clean-up step can be performed, as described below (e.g., exonuclease digestion, dilution of product, etc.). A second round of preamplification can then be carried out in which an inner "nester" reverse primer targets the sequence between (or the overlap of) the first two primers. This gives a third level of specificity. The reverse primer may, optionally, have a tag attached.

The same considerations that apply to a single round of preamplication, as discussed above, also apply to a second round of preamplification.

An advantage of the semi-nested approach is that very short target sequences can be amplified. In extremis, the primers of the first round of preamp may even overlap by 1, 2 or 3 nucleotides. An advantage of the fully nested approach (two outer primers and two inner primers) is that all four primers used are target specific. In order to obtain the correct PCR product and use non-target-specific detection methods (e.g., nucleotide tag-specific detection) in downstream analysis (e.g., in digital PCR), this is a useful improvement.

### Use of DNA-Binding Dye to Increase Melting Temperature of Amplification Target or Non-Target Nucleic Acid Sequences

DNA-binding dyes (e.g., intercalating dyes) such as Eva Green are known to increase the melting temperature of double-stranded DNA. This effect is more pronounced in GC rich sequences. Therefore, by using Eva Green or other such dyes one can bias amplification against GC rich amplicons.

To enrich for short sequences, nucleic acids in/from a biological sample can be circularized by ligation, e.g., using circligase. Short nucleic acid fragments will be circularized with higher efficiency than long fragments. Non-circularized sequences can be separated from circularized sequences, and the enriched short target nucleic acids used for further analysis.

In certain embodiments, ligation can be employed to add adapters to both ends of a nucleic acid fragment. Using these adapters, short fragments are actually enriched. Adaptors and ligation can be employed in a way that promotes circularization of nucleic acid fragments. Alternatively the end-product can be a circular double stranded DNA that still contains one or more nicks (preferably in the adaptor part) that can be used to open the circle using denaturation instead of an additional enzymatic step.

Circularization will be more efficient than ligating adaptors to both ends. Also, circularization introduces two distinct sequences to both ends of the nucleic acid fragment. In the simplest embodiment they are perfect complements. One can also choose to introduce mismatches. Alternatively, the adaptor can consist of more than two oligonucleotides.

### Transposase-Based Reduction of Longer DNA from Nucleic Acid Mixtures with a Large Size Distribution

In particular embodiments, the invention provides a method for enriching shorter fragments of nucleic acid (e.g., DNA) by selective tagging of longer nucleic acid fragments and subsequently removing them from solution by affinity purification methods directed to nucleic acid fragment ends inserted by a transposase.

Transposase is an enzyme that binds to the ends of a transposon and catalyzes the movement of the transposon to another part of the genome by a "cut and paste" mechanism or a replicative transposition mechanism.

The word "transposase" was first coined by the individuals who cloned the enzyme required for tranposition of the Tn3 transposon. Transposomes are formed when a transposase combines with a transposon (which contains specific DNA sequences, transposon ends, required for recognition by the transposase, and which are combined with the target DNA). Transposases have been engineered to insert transposon ends randomly in double stranded DNA. These transposases are available commercially (e.g., Epicentre EZ-Tn5).

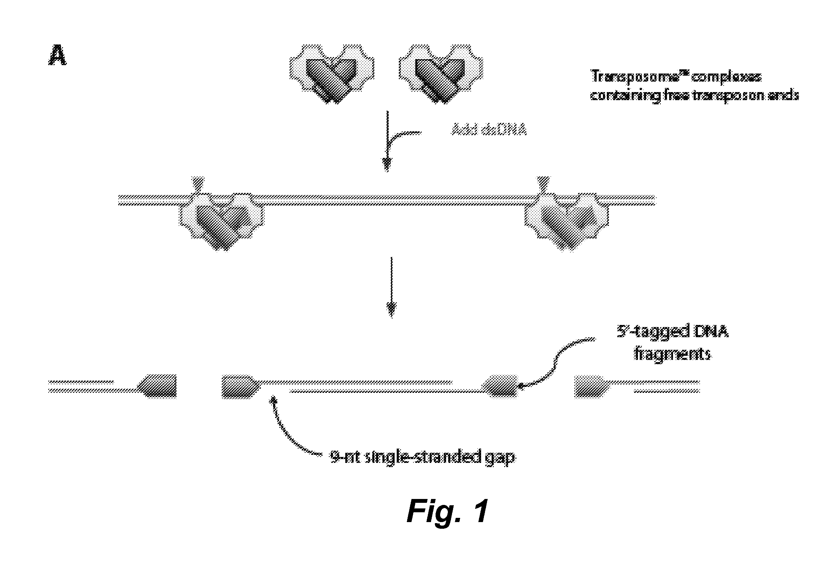

During *in vitro* transposition with hyperactive transposomes, strand-transfer occurs via random, staggered, double-stranded DNA breaks in the target DNA and covalent attachment of the 3'end of the transferred (top) transposon strand to the 5' end of the target DNA. When free transposon ends are used in the reaction, the target DNA is fragmented and the transferred strand of the transposon end oligonucleotide is covalently attached to the 5'end of the fragment (Figure 1). The size distribution of the fragments can be controlled by changing the amount of transposomes and reaction buffer conditions.

Genomic DNA can be fragmented to <1 kb in 5 minutes with enhanced reaction conditions. After the transposase reaction is finished, DNA fragments are tagged with the transposon ends. These transposon ends can be used as affinity tags to remove transposon-tagged DNA from the nucleic acid mixture. The transposon ends could be modified with tags known to those skilled in the art, including, but not limited to, for example, biotin, digoxigenin, specific nucleic acid sequences for hybridization. Transposase reaction conditions can be developed in which only longer dsDNA fragments (e.g. >200bp) undergo transposon end insertion. This can be achieved by either engineering the transposase to work with fragments of a particular length or by modifiying reaction conditions (number of transposases, concentration of magnesium ions).

Selective capture of longer nucleic acid fragments can be achieved by transposase-mediated tagging of a DNA sample followed by removal of transposon-tagged fragments by affinity-based methods specific to the transposon-ends.

### Sample Nucleic Acids

Preparations of nucleic acids ("samples") can be obtained from biological sources and prepared using conventional methods known in the art. In particular, DNA or RNA useful in the methods described herein can be extracted and/or amplified from any source, including bacteria, protozoa, fungi, viruses, organelles, as well higher organisms such as plants or animals, particularly mammals, and more particularly humans. Suitable nucleic acids can also be obtained from environmental sources (e.g., pond water), from man-made products (e.g., food), from forensic samples, and the like. Nucleic acids can be extracted or amplified from cells, bodily fluids (e.g., blood, a blood fraction, urine, etc.), or tissue samples by any of a variety of standard techniques. Illustrative samples include samples of plasma, serum, spinal fluid, lymph fluid, peritoneal fluid, pleural fluid, oral fluid, and external sections of the skin; samples from the respiratory, intestinal genital, and urinary tracts; samples of tears, saliva, blood cells, stem cells, or tumors. For example, samples of fetal DNA can be obtained from an embryo or from maternal blood, cervico-vaginal secretions, or urine. Any of these samples can, in certain embodiments, be analyzed or treated to enrich for short nucleic acids without prior fractionation.

In specific embodiments, the sample includes a sample of a maternal bodily fluid, or a fraction thereof, from a pregnant subject. For example, samples of whole blood, plasma, urine, and/or cervico-vaginal secretions can be employed in the methods described herein

Nucleic acids of interest can be isolated using methods well known in the art, with the choice of a specific method depending on the source, the nature of nucleic acid, and similar factors. The sample nucleic acids need not be in pure form, but are typically sufficiently pure to allow the amplification steps of the methods of the invention to be performed. Where the target nucleic acids are RNA, the RNA can be reversed transcribed into cDNA by standard methods known in the art and as described in Sambrook, J., Fritsch, E.F., and Maniatis, T., Molecular Cloning: A Laboratory Manual. Cold Spring Harbor Laboratory Press, NY, Vol. 1, 2, 3 (1989), for example. The cDNA can then be analyzed according to the methods of the invention.

### Target Nucleic Acids

Any target nucleic acid that can be tagged in an encoding reaction of the invention (described herein) can be detected using the methods of the invention. In typical embodiments, at least some nucleotide sequence information will be known for the target nucleic acids. For example, if the encoding reaction employed is PCR, sufficient sequence information is generally available for each end of a given target nucleic acid to permit design of suitable amplification primers. In an alternative embodiment, the target-specific sequences in primers could be replaced by random or degenerate nucleotide sequences.

The targets can include, for example, nucleic acids associated with pathogens, such as viruses, bacteria, protozoa, or fungi; RNAs, e.g., those for which over- or under-expression is indicative of disease, those that are expressed in a tissue- or developmental-specific manner; or those that are induced by particular stimuli; genomic DNA, which can be analyzed for specific polymorphisms (such as SNPs), alleles, or haplotypes, e.g., in genotyping. Of particular interest are genomic DNAs that are altered (e.g., amplified, deleted, and/or mutated) in genetic diseases or other pathologies; sequences that are associated with desirable or undesirable traits; and/or sequences that uniquely identify an individual (e.g., in forensic or paternity determinations).

In specific embodiments, at least some of the target amplicons, alleles, target nucleic acids, or loci analyzed according to the methods herein are derived from, or include fetal, DNA. For example, the sample to be analyzed can include a sample of a maternal bodily fluid, such as blood, or a fraction thereof, and at least some of the target nucleic acids can include fetal DNA.

### Primer Design

Primers suitable for nucleic acid amplification are sufficiently long to prime the synthesis of extension products in the presence of the agent for polymerization. The exact length and composition of the primer will depend on many factors, including, for example, temperature of the annealing reaction, source and composition of the primer, and where a probe is employed, proximity of the probe annealing site to the primer annealing site and ratio of primer:probe concentration. For example, depending on the complexity of the target nucleic acid sequence, an oligonucleotide primer typically contains in the range of about 15 to about 30 nucleotides, although it may contain more or fewer nucleotides. The primers should be sufficiently complementary to selectively anneal to their respective strands and form stable duplexes. One skilled in the art knows how to select appropriate primer pairs to amplify the target nucleic acid of interest.

For example, PCR primers can be designed by using any commercially available software or open source software, such as Primer3 (*see, e.g.,* Rozen and Skaletsky (2000) Meth. Mol. Biol., 132: 365-386; www.broad.mit.edu/node/1060, and the like) or by accessing the Roche UPL website. The amplicon sequences are input into the Primer3 program with the UPL probe sequences in brackets to ensure that the Primer3 program will design primers on either side of the bracketed probe sequence.

In certain embodiments, primers including nucleotide tags can be designed so that they form a stem-loop structure to avoid increased mis-hybridization because of nucleotide tag. In some embodiments, a nucleotide tag can be blocked by a complementary oligonucleotide that binds to it during the annealing step to prevent the nucleotide tag from contributing to non-specific hybridization and mis-priming.

Primers may be prepared by any suitable method, including, for example, cloning and restriction of appropriate sequences or direct chemical synthesis by methods such as the phosphotriester method of Narang et al. (1979) Meth. Enzymol. 68: 90-99; the phosphodiester method of Brown et al. (1979) Meth. Enzymol. 68: 109-151; the diethylphosphoramidite method of Beaucage et al. (1981) Tetra. Lett., 22: 1859-1862; the solid support method of U.S. Patent No. 4,458,066 and the like, or can be provided from a commercial source.

Primers may be purified by using a Sephadex column (Amersham Biosciences, Inc., Piscataway, NJ) or other methods known to those skilled in the art. Primer purification may improve the sensitivity of the methods of the invention.

### Quantitative Real-Time PCR and Other Detection and Quantification Methods

Any method of detection and/or quantification of nucleic acids can be used in the invention to detect amplification products. In one embodiment, PCR (polymerase chain reaction) is used to amplify and/or quantify target nucleic acids. In other embodiments, other amplification systems or detection systems are used, including, e.g., systems described in U.S. Pat. No. 7,118,910 and Invader assays; PE BioSystems). In particular embodiments, real-time quantification methods are used. For example, "quantitative real-time PCR" methods can be used to determine the quantity of a target nucleic acid present in a sample by measuring the amount of amplification product formed during the amplification process itself.

Fluorogenic nuclease assays are one specific example of a real-time quantification method that can be used successfully in the methods described herein. This method of monitoring the formation of amplification product involves the continuous measurement of PCR product accumulation using a dual-labeled fluorogenic oligonucleotide probe--an approach frequently referred to in the literature as the "TaqMan® method." See U.S. Pat. No. 5,723,591; Heid et al., 1996, Real-time quantitative PCR Genome Res. 6:986-94. It will be appreciated that while "TaqMan® probes" are the most widely used for qPCR, the invention is not limited to use of these probes; any suitable probe can be used.

Other detection/quantification methods that can be employed in the present invention include FRET and template extension reactions, molecular beacon detection, Scorpion detection, Invader detection, and padlock probe detection.

FRET and template extension reactions utilize a primer labeled with one member of a donor/acceptor pair and a nucleotide labeled with the other member of the donor/acceptor pair. Prior to incorporation of the labeled nucleotide into the primer during a template-dependent extension reaction, the donor and acceptor are spaced far enough apart that energy transfer cannot occur. However, if the labeled nucleotide is incorporated into the primer and the spacing is sufficiently close, then energy transfer occurs and can be detected. These methods are particularly useful in conducting single base pair extension reactions in the detection of single nucleotide polymorphisms and are described in U.S. Patent No. 5,945,283 and PCT Publication WO 97/22719.

With molecular beacons, a change in conformation of the probe as it hybridizes to a complementary region of the amplified product results in the formation of a detectable signal. The probe itself includes two sections: one section at the 5' end and the other section at the 3' end. These sections flank the section of the probe that anneals to the probe binding site and are complementary to one another. One end section is typically attached to a reporter dye and the other end section is usually attached to a quencher dye. In solution, the two end sections can hybridize with each other to form a hairpin loop. In this conformation, the reporter and quencher dye are in sufficiently close proximity that fluorescence from the reporter dye is effectively quenched by the quencher dye. Hybridized probe, in contrast, results in a linearized conformation in which the extent of quenching is decreased. Thus, by monitoring emission changes for the two dyes, it is possible to indirectly monitor the formation of amplification product. Probes of this type and methods of their use are described further, for example, by Piatek et al., 1998, Nat. Biotechnol. 16:359-63; Tyagi, and Kramer, 1996, Nat. Biotechnology 14:303-308; and Tyagi, et al., 1998, Nat. Biotechnol. 16:49-53 (1998).

The Scorpion detection method is described, for example, by Thelwell et al. 2000, Nucleic Acids Research, 28:3752-3761 and Solinas et al., 2001, "Duplex Scorpion primers in SNP analysis and FRET applications" Nucleic Acids Research 29:20. Scorpion primers are fluorogenic PCR primers with a probe element attached at the 5'-end via a PCR stopper. They are used in real-time amplicon-specific detection of PCR products in homogeneous solution. Two different formats are possible, the "stem-loop" format and the "duplex" format. In both cases the probing mechanism is intramolecular. The basic elements of Scorpions in all formats are: (i) a PCR primer; (ii) a PCR stopper to prevent PCR read-through of the probe element; (iii) a specific probe sequence; and (iv) a fluorescence detection system containing at least one fluorophore and quencher. After PCR extension of the Scorpion primer, the resultant amplicon contains a sequence that is complementary to the probe, which is rendered single-stranded during the denaturation stage of each PCR cycle. On cooling, the probe is free to bind to this complementary sequence, producing an increase in fluorescence, as the quencher is no longer in the vicinity of the fluorophore. The PCR stopper prevents undesirable read-through of the probe by Taq DNA polymerase.

Invader assays (Third Wave Technologies, Madison, WI) are used particularly for SNP genotyping and utilize an oligonucleotide, designated the signal probe, that is complementary to the target nucleic acid (DNA or RNA) or polymorphism site. A second oligonucleotide, designated the Invader Oligo, contains the same 5' nucleotide sequence, but the 3' nucleotide sequence contains a nucleotide polymorphism. The Invader Oligo interferes with the binding of the signal probe to the target nucleic acid such that the 5' end of the signal probe forms a "flap" at the nucleotide containing the polymorphism. This complex is recognized by a structure specific endonuclease, called the Cleavase enzyme. Cleavase cleaves the 5' flap of the nucleotides. The released flap binds with a third probe bearing FRET labels, thereby forming another duplex structure recognized by the Cleavase enzyme. This time, the Cleavase enzyme cleaves a fluorophore away from a quencher and produces a fluorescent signal. For SNP genotyping, the signal probe will be designed to hybridize with either the reference (wild type) allele or the variant (mutant) allele. Unlike PCR, there is a linear amplification of signal with no amplification of the nucleic acid. Further details sufficient to guide one of ordinary skill in the art are provided by, for example, Neri, B.P., et al., Advances in Nucleic Acid and Protein Analysis 3826:117-125, 2000) and U.S. Patent No. 6,706,471.

Padlock probes (PLPs) are long (e.g., about 100 bases) linear oligonucleotides. The sequences at the 3' and 5' ends of the probe are complementary to adjacent sequences in the target nucleic acid. In the central, noncomplementary region of the PLP there is a "tag" sequence that can be used to identify the specific PLP. The tag sequence is flanked by universal priming sites, which allow PCR amplification of the tag. Upon hybridization to the target, the two ends of the PLP oligonucleotide are brought into close proximity and can be joined by enzymatic ligation. The resulting product is a circular probe molecule catenated to the target DNA strand. Any unligated probes (i.e., probes that did not hybridize to a target) are removed by the action of an exonuclease. Hybridization and ligation of a PLP requires that both end segments recognize the target sequence. In this manner, PLPs provide extremely specific target recognition.

The tag regions of circularized PLPs can then be amplified and resulting amplicons detected. For example, TaqMan® real-time PCR can be carried out to detect and quantify the amplicon. The presence and amount of amplicon can be correlated with the presence and quantity of target sequence in the sample. For descriptions of PLPs see, e.g., Landegren et al., 2003, Padlock and proximity probes for in situ and array-based analyses: tools for the post-genomic era, Comparative and Functional Genomics 4:525-30; Nilsson et al., 2006, Analyzing genes using closing and replicating circles Trends Biotechnol. 24:83-8; Nilsson et al., 1994, Padlock probes: circularizing oligonucleotides for localized DNA detection, Science 265:2085-8.

In particular embodiments, fluorophores that can be used as detectable labels for probes include, but are not limited to, rhodamine, cyanine 3 (Cy 3), cyanine 5 (Cy 5), fluorescein, Vic™, Liz™., Tamra™, 5-Fam™, 6-Fam™, and Texas Red (Molecular Probes). (Vic™, Liz™, Tamra™, 5-Fam™, 6-Fam™ are all available from Applied Biosystems, Foster City, Calif.).

Devices have been developed that can perform a thermal cycling reaction with compositions containing a fluorescent indicator, emit a light beam of a specified wavelength, read the intensity of the fluorescent dye, and display the intensity of fluorescence after each cycle. Devices comprising a thermal cycler, light beam emitter, and a fluorescent signal detector, have been described, e.g., in U.S. Pat. Nos. 5,928,907; 6,015,674; and 6,174,670.

In some embodiments, each of these functions can be performed by separate devices. For example, if one employs a Q-beta replicase reaction for amplification, the reaction may not take place in a thermal cycler, but could include a light beam emitted at a specific wavelength, detection of the fluorescent signal, and calculation and display of the amount of amplification product.

In particular embodiments, combined thermal cycling and fluorescence detecting devices can be used for precise quantification of target nucleic acids. In some embodiments, fluorescent signals can be detected and displayed during and/or after one or more thermal cycles, thus permitting monitoring of amplification products as the reactions occur in "real-time." In certain embodiments, one can use the amount of amplification product and number of amplification cycles to calculate how much of the target nucleic acid sequence was in the sample prior to amplification.

According to some embodiments, one can simply monitor the amount of amplification product after a predetermined number of cycles sufficient to indicate the presence of the target nucleic acid sequence in the sample. One skilled in the art can easily determine, for any given sample type, primer sequence, and reaction condition, how many cycles are sufficient to determine the presence of a given target nucleic acid.

By acquiring fluorescence over different temperatures, it is possible to follow the extent of hybridization. Moreover, the temperature-dependence of PCR product hybridization can be used for the identification and/or quantification of PCR products. Accordingly, the methods described herein encompass the use of melting curve analysis in detecting and/or quantifying amplicons. Melting curve analysis is well known and is described, for example, in U.S. Patent Nos. 6,174,670; 6472156; and 6,569,627. In illustrative embodiments, melting curve analysis is carried out using a double-stranded DNA dye, such as SYBR Green, Eva Green, Pico Green (Molecular Probes, Inc., Eugene, OR), ethidium bromide, and the like (see Zhu et al., 1994, Anal. Chem. 66:1941-48).

According to certain embodiments, one can employ an internal control to quantify the amplification product indicated by the fluorescent signal. See, e.g., U.S. Pat. No. 5,736,333.

In various embodiments, employing preamplification, the number of preamplification cycles is sufficient to add one or more nucleotide tags to the target nucleotide sequences, so that the relative copy numbers of the tagged target nucleotide sequences is substantially representative of the relative copy numbers of the target nucleic acids in the sample. For example, preamplification can be carried out for 2-20 cycles to introduce the sample-specific or set-specific nucleotide tags. In other embodiments, detection is carried out at the end of exponential amplification, i.e., during the "plateau" phase, or endpoint PCR is carried out. In this instance, preamplification will normalize amplicon copy number across targets and across samples. In various embodiments, preamplification and/or amplification can be carried out for about: 2, 4, 10, 15, 20, 25, 30, 35, or 40 cycles or for a number of cycles falling within any range bounded by any of these values.

### Digital Amplification

For discussions of "digital PCR" see, for example, Vogelstein and Kinzler, 1999, Proc Natl Acad Sci USA 96:9236-41; McBride et al., U.S Patent Application Publication No. 20050252773, especially Example 5. Digital amplification methods can make use of certain-high-throughput devices suitable for digital PCR, such as microfluidic devices typically including a large number and/or high density of small-volume reaction sites (e.g., nano-volume reaction sites or reaction chambers). In illustrative embodiments, digital amplification is performed using a matrix-type microfluidic device, such as the Digital Array™ microfluidic devices described below. Digital amplification can entail distributing or partitioning a sample among hundreds to thousands of reaction mixtures. These reaction mixtures can be disposed in a reaction/assay platform or microfluidic device or can exist as separate droplets, e.g, as in emulsion PCR. Methods for creating droplets having reaction component(s) and/or conducting reactions therein are described in U.S. Patent No. 7,294,503, issued to Quake et al. U.S. Patent Publication No. 20100022414, published January 28, 2010 (assigned to Raindance Technologies, Inc.) U.S. Patent Publication No. 20100092973, published on April 15, 2010 (assigned to Stokes Bio Ltd.). In such embodiments, a limiting dilution of the sample is made across a large number of separate amplification reactions such that most of the reactions have no template molecules and give a negative amplification result. In counting the number of positive amplification results, e.g, at the reaction endpoint, one is counting the individual template molecules present in the original sample one-by-one. A major advantage of digital amplification is that the quantitation is independent of variations in the amplification efficiency - successful amplifications are counted as one molecule, independent of the actual amount of product.

In particular embodiments, the methods of the invention are employed in determining the copy number of one or more target nucleic acids in a nucleic acid sample. In specific embodiments, methods and systems described herein can be used to detect copy number variation of a target nucleic acid in the genome of a subject by analyzing the genomic DNA present in a sample derived from the subject. For example, digital amplification can be carried out to determine the relative number of copies of a target nucleic acid and a reference nucleic acid in a sample. In certain embodiments, the genomic copy number is known for the reference nucleic acid (i.e., known for the particular nucleic acid sample under analysis). Alternatively, the reference nucleic acid can be one that is normally present in two copies (and unlikely to be amplified or deleted) in a diploid genome, and the copy number in the nucleic acid sample being analyzed is assumed to be two. For example, useful reference nucleic acids in the human genome include sequences of the RNaseP, ß-actin, and glyceraldehyde-3-phosphate dehydrogenase (GAPDH) genes; however, it will be appreciated the invention is not limited to a particular reference nucleic acid.

In certain embodiments, digital amplification can be carried out after preamplification of sample nucleic acids. Typically, preamplification prior to digital amplification is performed for a limited number of thermal cycles (e.g., 5 cycles, or 10 cycles). In certain embodiments, the number of thermal cycles during preamplification can range from about 4 to 15 thermal cycles, or about 4-10 thermal cycles. In specific embodiments the number of thermal cycles can be 3, 4, 5, 6, 7, 8, 9, 10, 11, 12, 13, 14, 15, or more than 15. As those of skill in the art will appreciate, two or more cycles of the tagging amplification methods described above is sufficient to produce tagged target nucleotide sequence(s). When performing digital amplification for copy number determination, at least one target nucleotide sequence and at least one reference nucleotide sequence can be tagged. In certain embodiments, this amplification can be continued for a suitable number of cycles for a typical preamplification step, rendering a separate preamplification step unnecessary. Alternatively, different primers, such as, for example, tag-specific primers could be contacted with the tagged target and reference nucleotide sequences and preamplification carried out. For ease of discussion, the term "preamplification" is used below to describe amplification performed prior to digital amplification and the products of this amplification are termed "amplicons."

In particular embodiments, preamplification reactions preferably provide quantitative amplification of the nucleic acids in the reaction mixture. That is, the relative number (ratio) of the target and reference amplicons should reflect the relative number (ratio) of target and reference nucleic acids in the nucleic acids being amplified. Methods for quantitative amplification are known in the art. See, e.g., Arya et al., 2005, Basic principles of real-time quantitative PCR, Expert Rev Mol Diagn. 5(2):209-19. In general, primer pairs and preamplification conditions can be selected to ensure that the amplification efficiencies tagged target and tagged reference nucleotide sequences are similar or approximately equal, in order reduce any bias in the copy number determination. The amplification efficiency of any pair of primers can be easily determined using routine techniques (see e.g., Furtado et al., "Application of real-time quantitative PCR in the analysis of gene expression." DNA amplification: Current Technologies and Applications. Wymondham, Norfolk, UK: Horizon Bioscience p. 131-145 (2004)). If the target and reference nucleotide sequences are tagged with the same tags, under suitable conditions, tag-specific primers can amplify both target and reference nucleotide sequences with similar or approximately equal amplification efficiencies. Further, limiting the number of preamplification cycles (typically to less than 15, usually 10 or less than 10, more usually about 5) greatly mitigates any differences in efficiency, such that the typical differences are likely to have an insignificant effect on the results.

Thus, following preamplification and distribution of the preamplified target and reference amplicons into separate digital amplification mixtures, a proportional number of amplicons corresponding to each sequence will be distributed into the mixtures. After digital amplification, the ratio of target and reference amplification products reflects the original ratio. Therefore, one can determine the number of reaction mixtures containing amplification product derived from the target amplicon and determine the number of reaction mixtures containing amplification product derived from the reference amplicon; and the ratio of these numbers provides the copy number of the target nucleic acid (e.g., the tagged target nucleotide sequence) relative to the reference nucleic acid (e.g., the tagged reference nucleotide sequence).

Generally, in digital amplification, identical (or substantially similar) amplification reactions are run on a nucleic acid sample, such as genomic DNA. The number of individual reactions for a given nucleic acid sample may vary from about 2 to over 1,000,000. Typically, the number of reactions performed on a sample is about 100 or greater, more typically about 200 or greater, and even more typically about 300 or greater. Larger scale digital amplification can also be performed in which the number of reactions performed on a sample is about 500 or greater, about 700 or greater, about 765 or greater, about 1,000 or greater, about 2,500 or greater, about 5,000 or greater, about 7,500 or greater, or about 10,000or greater. The number of reactions performed may also be significantly higher, such up to about 25,000, up to about 50,000, up to about 75,000, up to about 100,000, up to about 250,000, up to about 500,000, up to about 750,000, up to about 1,000,000, or even greater than 1,000,000 assays per genomic sample.

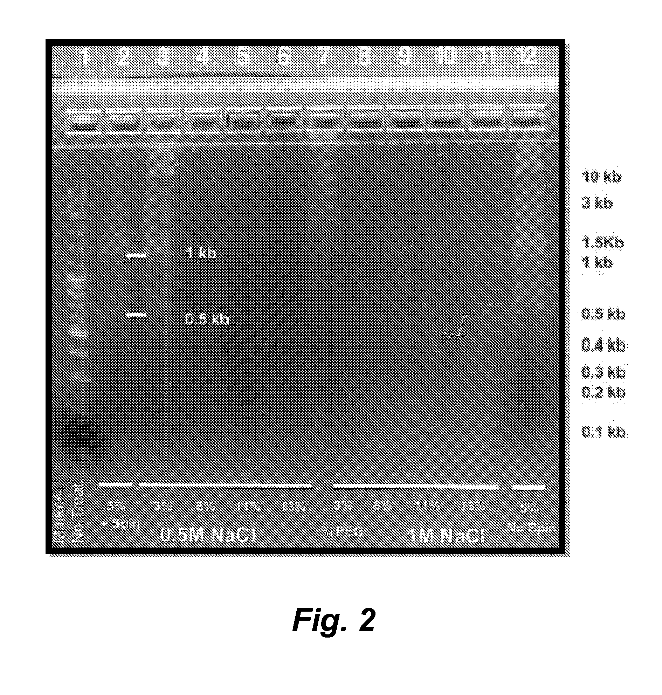

In particular embodiments, the quantity of nucleic acid subjected to digital amplification is generally selected such that, when distributed into discrete reaction mixtures, each individual amplification reaction is expected to include one or fewer amplifiable nucleic acids. One of skill in the art can determine the concentration of target amplicon(s) produced as described above and calculate an appropriate amount for use in digital amplification. More conveniently, a set of serial dilutions of the target amplicon(s) can be tested. For example, the device shown in Figure 2 (commercially available from Fluidigm Corp. as the 12.765 Digital Array™ IFC) allows 12 different dilutions to be tested simultaneously. Optionally, a suitable dilution can be determined by generating a linear regression plot. For the optimal dilution, the line should be straight and pass through the origin. Subsequently the concentration of the original samples can be calculated from the plot.

The appropriate quantity of target and reference amplicon(s) can be distributed into discrete locations or reaction wells or chambers such that each reaction includes, for example, an average of no more than about one target amplicon and one reference amplicon per volume. The target and reference amplicon(s) can be combined with reagents selected for quantitative or nonquantitative amplification, prior to distribution or after.

Following distribution, the reaction mixtures are subjected to amplification to identify those reaction mixtures that contain a target and/or amplicon. Any amplification method can be employed, but conveniently, PCR is used, e.g., real-time PCR or endpoint PCR. This amplification can employ any primers capable of amplifying the target and/or reference amplicon(s). Digital amplification can be can be carried out wherein the target and reference amplicons are distributed into sets of reaction mixtures for detection of amplification products derived from one type of amplicon, either target or reference amplicons. In such embodiments, two sets of reaction mixtures, a target set and a reference set, could have distinct primer pairs, one for amplifying target amplicons, and one for amplifying reference amplicons could be used. Amplification product could be detected, for example, using a universal probe, such as SYBR Green, or target- and reference-specific probes, which could be included in all digital amplification mixtures.

The concentration of any target or reference amplicon (copies/µL) is correlated with the number of reaction mixtures that are positive (i.e., amplification product-containing) for that particular amplicon. See copending U.S. Application No. 12/170,414, entitled "Method and Apparatus for Determining Copy Number Variation Using Digital PCR,". Also see Dube et al., 2008, "Mathematical Analysis of Copy Number Variation in a DNA Sample Using Digital PCR on a Nanofluidic Device" PLoS ONE 3(8): e2876. doi:10.1371/journal.pone.0002876.

### DNA Sequencing

Many current DNA sequencing techniques rely on "sequencing by synthesis." These techniques entail library creation, massively parallel PCR amplification of library molecules, and sequencing. Library creation starts with conversion of sample nucleic acids to appropriately sized fragments, ligation of adaptor sequences onto the ends of the fragments, and selection for molecules properly appended with adaptors. The presence of the adaptor sequences on the ends of the library molecules enables amplification of random-sequence inserts. The above-described methods for tagging nucleotide sequences can be substituted for ligation, to introduce adaptor sequences.

In particular embodiments, the number of library DNA molecules produced in the massively parallel PCR step is low enough that the chance of two molecules associating with the same substrate, e.g. the same bead (in 454 DNA sequencing) or the same surface patch (in Solexa DNA sequencing) is low, but high enough so that the yield of amplified sequences is sufficient to provide a high throughput. After suitable adaptor sequences are introduced, digital PCR can be employed to calibrate the number of library DNA molecules prior to sequencing by synthesis.

The methods of the invention can include subjecting at least one target amplicon to DNA sequencing using any available DNA sequencing method. In particular embodiments, a plurality of target amplicons is sequenced using a high throughput sequencing method. Such methods typically use an in vitro cloning step to amplify individual DNA molecules. Emulsion PCR (emPCR) isolates individual DNA molecules along with primer-coated beads in aqueous droplets within an oil phase. PCR produces copies of the DNA molecule, which bind to primers on the bead, followed by immobilization for later sequencing. emPCR is used in the methods by Marguilis et al. (commercialized by 454 Life Sciences, Branford, CT), Shendure and Porreca et al. (also known as "polony sequencing") and SOLiD sequencing, (Applied Biosystems Inc., Foster City, CA). See M. Margulies, et al. (2005) "Genome sequencing in microfabricated high-density picolitre reactors" Nature 437: 376-380; J. Shendure, et al. (2005) "Accurate Multiplex Polony Sequencing of an Evolved Bacterial Genome" Science 309 (5741): 1728-1732. In vitro clonal amplification can also be carried out by "bridge PCR," where fragments are amplified upon primers attached to a solid surface. Braslavsky et al. developed a single-molecule method (commercialized by Helicos Biosciences Corp., Cambridge, MA) that omits this amplification step, directly fixing DNA molecules to a surface. I. Braslavsky, et al. (2003) "Sequence information can be obtained from single DNA molecules" Proceedings of the National Academy of Sciences of the United States of America 100: 3960-3964.

DNA molecules that are physically bound to a surface can be sequenced in parallel. "Sequencing by synthesis," like dye-termination electrophoretic sequencing, uses a DNA polymerase to determine the base sequence. Reversible terminator methods (commercialized by Illumina, Inc., San Diego, CA and Helicos Biosciences Corp., Cambridge, MA) use reversible versions of dye-terminators, adding one nucleotide at a time, and detect fluorescence at each position in real time, by repeated removal of the blocking group to allow polymerization of another nucleotide. "Pyrosequencing" also uses DNA polymerization, adding one nucleotide at a time and detecting and quantifying the number of nucleotides added to a given location through the light emitted by the release of attached pyrophosphates (commercialized by 454 Life Sciences, Branford, CT). See M. Ronaghi, et al. (1996). "Real-time DNA sequencing using detection of pyrophosphate release" Analytical Biochemistry 242: 84-89.

### Labeling Strategies

Any suitable labeling strategy can be employed in the methods of the invention. Where the assay mixture is aliquoted, and each aliquot is analyzed for presence of a single amplification product, a universal detection probe can be employed in the amplification mixture. In particular embodiments, real-time PCR detection can be carried out using a universal qPCR probe. Suitable universal qPCR probes include double-stranded DNA dyes, such as SYBR Green, Pico Green (Molecular Probes, Inc., Eugene, OR), Eva Green (Biotinum), ethidium bromide, and the like (see Zhu et al., 1994, Anal. Chem. 66:1941-48). Suitable universal qPCR probes also include sequence-specific probes that bind to a nucleotide sequence present in all amplification products. Binding sites for such probes can be conveniently introduced into the tagged target nucleic acids during amplification.

Alternatively, one or more target-specific qPCR probes (i.e., specific for a target nucleotide sequence to be detected) is employed in the amplification mixtures to detect amplification products. Target-specific probes could be useful, e.g., when only a few target nucleic acids are to be detected in a large number of samples. For example, if only three targets were to be detected, a target-specific probe with a different fluorescent label for each target could be employed. By judicious choice of labels, analyses can be conducted in which the different labels are excited and/or detected at different wavelengths in a single reaction. See, e.g., Fluorescence Spectroscopy (Pesce et al., Eds.) Marcel Dekker, New York, (1971); White et al., Fluorescence Analysis: A Practical Approach, Marcel Dekker, New York, (1970); Berlman, Handbook of Fluorescence Spectra of Aromatic Molecules, 2nd ed., Academic Press, New York, (1971); Griffiths, Colour and Constitution of Organic Molecules, Academic Press, New York, (1976); Indicators (Bishop, Ed.). Pergamon Press, Oxford, 19723; and Haugland, Handbook of Fluorescent Probes and Research Chemicals, Molecular Probes, Eugene (1992).

An "indirect" labeling strategy can be employed wherein the amplicon to be detection includes a nucleotide tag or when a primer in a preamplification or amplification mixture includes such a tag. In this case, an amplification mixture can included a labeled (e.g., fluorescently labeled) nucleotide tag-specific primer.

Other labeling strategies that can be employed in the methods described herein include, e.g., that described in U.S. Patent No. 7,615,620, issued November 10, 2009 to Robinson (assigned to KBiosciences Ltd.), which discloses a FRET detection system for an amplification process that employs at least two single-labeled oligonucleotide sequences of differing Tm that hybridize to one another in free solution to form a fluorescent quenched pair, that upon introduction of a complementary sequence to one or both sequences generates a measurable signal, one of the sequences being of a Tm that is below the Ta of the PCR process, the other not being below the Ta of the PCR process. This patent is incorporated herein in its entirety and for this disclosure.

International Publication No. WO/1997/032044, published September 4, 1997 (assigned to E.I. Du Pont De Nemours And Company) describes a detection probe the is present throughout an amplification reaction but does not participate in the reaction in that it is not extended. The probe contains sequence complementary to the replicated nucleic acid target for capture of the target by hybridization. Additionally, the probe or target contains at least one reactive ligand to permit immobilization or reporting of the probe/target hybrid. Such labeling systems can be employed in the methods described herein.

Additional labeling strategies useful in the methods described herein are found in U.S. Patent No. 5,928,862, issued July 27, 1999 to Morrison (assigned to Amoco Corp.), which discloses a competitive homogeneous assay.

U.S. Patent No. 6,103,476, issued August 15, 2000 to Tyagi et al. (assigned to The Public Health Research Institute of the City of New York, Inc.) describes unimolecular and bimolecular hybridization probes that include a target complement sequence, an affinity pair holding the probe in a closed conformation in the absence of target sequence, and either a label pair that interacts when the probe is in the closed conformation or, for certain unimolecular probes, a non-interactive label. Hybridization of the target and target complement sequences shifts the probe to an open conformation. The shift is detectable due to reduced interaction of the label pair or by detecting a signal from a non-interactive label. Certain unimolecular probes can discriminate between target and non-target sequences differing by as little as one nucleotide. Such labeling systems can be employed in the methods described herein.

### Removal of Undesired Reaction Components

It will be appreciated that reactions involving complex mixtures of nucleic acids in which a number of reactive steps are employed can result in a variety of unincorporated reaction components, and that removal of such unincorporated reaction components, or reduction of their concentration, by any of a variety of clean-up procedures can improve the efficiency and specificity of subsequently occurring reactions. For example, it may be desirable, in some embodiments, to remove, or reduce the concentration of preamplification primers prior to carrying out the amplification steps described herein.

In certain embodiments, the concentration of undesired components can be reduced by simple dilution. For example, preamplified samples can be diluted about 2-, 5-, 10-, 50-, 100-, 500-, 1000-fold prior to amplification to improve the specificity of the subsequent amplification step.

In some embodiments, undesired components can be removed by a variety of enzymatic means. Alternatively, or in addition to the above-described methods, undesired components can be removed by purification. For example, a purification tag can be incorporated into any of the above-described primers to facilitate purification of the tagged target nucleotides.

In particular embodiments, clean-up includes selective immobilization of the desired nucleic acids. For example, desired nucleic acids can be preferentially immobilized on a solid support. In an illustrative embodiment, an affinity moiety, such as biotin (e.g., photo-biotin), is attached to desired nucleic acid, and the resulting biotin-labeled nucleic acids immobilized on a solid support comprising an affinity moiety-binder such as streptavidin. Immobilized nucleic acids can be queried with probes, and non-hybridized and/or non-ligated probes removed by washing (See, e.g., Published P.C.T. Application WO 03/006677 and USSN 09/931,285.) Alternatively, immobilized nucleic acids can be washed to remove other components and then released from the solid support for further analysis. This approach can be used, for example, in recovering target amplicons from amplification mixtures after the addition of primer binding sites for DNA sequencing. In particular embodiments, an affinity moiety, such as biotin, can be attached to an amplification primer such that amplification produces an affinity moiety-labeled (e.g., biotin-labeled) amplicon.

### Microfluidic Devices

In certain embodiments, any of the methods of the invention can be carried out using a microfluidic device. In illustrative embodiments, the device is a matrix-type microfluidic device is one that allows the simultaneous combination of a plurality of substrate solutions with reagent solutions in separate isolated reaction chambers. It will be recognized, that a substrate solution can comprise one or a plurality of substrates and a reagent solution can comprise one or a plurality of reagents. For example, the microfluidic device can allow the simultaneous pair-wise combination of a plurality of different amplification primers and samples. In certain embodiments, the device is configured to contain a different combination of primers and samples in each of the different chambers. In various embodiments, the number of separate reaction chambers can be greater than 50, usually greater than 100, more often greater than 500, even more often greater than 1000, and sometimes greater than 5000, or greater than 10,000.

In particular embodiments, the matrix-type microfluidic device is a Dynamic Array™ microfluidic device, an example of which is shown in Fig. 1. A Dynamic Array™ microfluidic device is a matrix-type microfluidic device designed to isolate pair-wise combinations of samples and reagents *(e.g.,* amplification primers, detection probes, *etc*.) and suited for carrying out qualitative and quantitative PCR reactions including real-time quantitative PCR analysis. In some embodiments, the DA microfluidic device is fabricated, at least in part, from an elastomer. DA microfluidic devices are described in PCT publication WO05107938A2 (Thermal Reaction Device and Method For Using The Same) and US Pat. Publication US20050252773A1. DA microfluidic devices may incorporate high-density matrix designs that utilize fluid communication vias between layers of the microfluidic device to weave control lines and fluid lines through the device and between layers. By virtue of fluid lines in multiple layers of an elastomeric block, high density reaction cell arrangements are possible. Alternatively DA microfluidic devices may be designed so that all of the reagent and sample channels are in the same elastomeric layer, with control channels in a different layer.

U.S. Patent Publication No. 2008/0223721 and PCT Publication No. WO 05/107938A2 describe illustrative matrix-type devices that can be used to practice the methods described herein. Figure 21 of the latter is reproduced as Fig. 1 and shows an illustrative matrix design having a first elastomeric layer 2110 (1st layer) and a second elastomeric layer 2120 (2d layer) each having fluid channels formed therein. For example, a reagent fluid channel in the first layer 2110 is connected to a reagent fluid channel in the second layer 2120 through a via 2130, while the second layer 2120 also has sample channels therein, the sample channels and the reagent channels terminating in sample and reagent chambers 2180, respectively. The sample and reagent chambers 2180 are in fluid communication with each other through an interface channel 2150 that has an interface valve 2140 associated therewith to control fluid communication between each of the chambers 2180 of a reaction cell 2160. In use, the interface is first closed, then reagent is introduced into the reagent channel from the reagent inlet and sample is introduced into the sample channel through the sample inlet; containment valves 2170 are then closed to isolate each reaction cell 2160 from other reaction cells 2160. Once the reaction cells 2160 are isolated, the interface valve 2140 is opened to cause the sample chamber and the reagent chamber to be in fluid communication with each other so that a desired reaction may take place. It will be apparent from this (and the description in WO 05/107938A2) that the DA microfluidic device may be used for reacting M number of different samples with N number of different reagents.

Although the DA microfluidic devices described above in WO 05/107938 are well suited for conducting the methods described herein, the invention is not limited to any particular device or design. Any device that partitions a sample and/or allows independent pair-wise combinations of reagents and sample may be used. U.S. Patent Publication No. 20080108063 includes a diagram illustrating the 48.48 Dynamic Array™ IFC (Integrated Fluidic Circuit), a commercially available device available from Fluidigm Corp. (South San Francisco Calf.). It will be understood that other configurations are possible and contemplated such as, for example, 48×96; 96×96; 30×120; *etc.*

In specific embodiments, the microfluidic device can be a Digital Array™ microfluidic device, which is adapted to perform digital amplification. Such devices can have integrated channels and valves that partition mixtures of sample and reagents into nanolitre volume reaction chambers. In some embodiments, the Digital Array™ microfluidic device is fabricated, at least in part, from an elastomer. Illustrative Digital Array™ microfluidic devices are described in copending U.S. Applications owned by Fluidigm, Inc., such as U.S. Application No. 12/170,414, entitled "Method and Apparatus for Determining Copy Number Variation Using Digital PCR." One illustrative embodiment has 12 input ports corresponding to 12 separate sample inputs to the device. The device can have 12 panels, and each of the 12 panels can contain 765 6 nL reaction chambers with a total volume of 4.59 µL per panel. Microfluidic channels can connect the various reaction chambers on the panels to fluid sources. Pressure can be applied to an accumulator in order to open and close valves connecting the reaction chambers to fluid sources. In illustrative embodiments, 12 inlets can be provided for loading of the sample reagent mixture. 48 inlets can be used to provide a source for reagents, which are supplied to the biochip when pressure is applied to accumulator. Additionally, two or more inlets can be provided to provide hydration to the biochip. Hydration inlets are in fluid communication with the device to facilitate the control of humidity associated with the reaction chambers. As will be understood to one of skill in the art, some elastomeric materials that can utilized in the fabrication of the device are gas permeable, allowing evaporated gases or vapor from the reaction chambers to pass through the elastomeric material into the surrounding atmosphere. In a particular embodiment, fluid lines located at peripheral portions of the device provide a shield of hydration liquid, for example, a buffer or master mix, at peripheral portions of the biochip surrounding the panels of reaction chambers, thus reducing or preventing evaporation of liquids present in the reaction chambers. Thus, humidity at peripheral portions of the device can be increased by adding a volatile liquid, for example water, to hydration inlets. In a specific embodiment, a first inlet is in fluid communication with the hydration fluid lines surrounding the panels on a first side of the biochip and the second inlet is in fluid communication with the hydration fluid lines surrounding the panels on the other side of the biochip.

While the Digital Array™ microfluidic devices are well-suited for carrying out the digital amplification methods described herein, one of ordinary skill in the art would recognize many variations and alternatives to these devices. The microfluidic device which is the 12.765 Dynamic Array™ IFC commercially available from Fluidigm Corp. (South San Francisco, CA), includes 12 panels, each having 765 reaction chambers with a volume of 6 nL per reaction chamber. However, this geometry is not required for the digital amplification methods described herein. The geometry of a given Digital Array™ microfluidic device will depend on the particular application. Additional description related to devices suitable for use in the methods described herein is provided in U.S. Patent Application Publication No. 2005/0252773.

In certain embodiments, the methods described herein can be performed using a microfluidic device that provides for recovery of reaction products. Such devices are described in detail in copending U.S. Application No. 61/166,105, filed April 2, 2009. For example, the digital PCR method for calibrating DNA samples prior to sequencing can be preformed on such devices, permitting recovery of amplification products, which can then serve as templates for DNA sequencing.

Another microfluidic device that can be employed in the methods described herein is disclosed in PCT Pub. No.WO/2009/059430, published 5/14/2009 (Hansen and Tropini). This microfluidic device includes a plurality of reaction chambers in fluid communication with a flow channel formed in an elastomeric substrate, a vapor barrier for preventing evaporation from the plurality of reaction chambers, and a continuous phase fluid for isolation of each of the plurality of reaction chambers.

Fabrication methods using elastomeric materials and methods for design of devices and their components have been described in detail in the scientific and patent literature. See, *e.g.,* Unger et al. (2000) Science 288:113-116; U.S. Pat. Nos. US 6,960,437 (Nucleic acid amplification utilizing microfluidic devices); 6,899,137 (Microfabricated elastomeric valve and pump systems); 6,767,706 (Integrated active flux microfluidic devices and methods); 6,752,922 (Microfluidic chromatography); 6,408,878 (Microfabricated elastomeric valve and pump systems); 6,645,432 (Microfluidic systems including three-dimensionally arrayed channel networks); U.S. Patent Application Publication Nos. 2004/0115838; 2005/0072946; 2005/0000900; 2002/0127736; 2002/0109114; 2004/0115838; 2003/0138829; 2002/0164816; 2002/0127736; and 2002/0109114; PCT Publication Nos. WO 2005/084191; WO 05/030822A2; and WO 01/01025; Quake & Scherer, 2000, "From micro to nanofabrication with soft materials" Science 290: 1536-40; Unger et al., 2000, "Monolithic microfabricated valves and pumps by multilayer soft lithography" Science 288:113-116; Thorsen et al., 2002, "Microfluidic large-scale integration" Science 298:580-584; Chou et al., 2000, "Microfabricated Rotary Pump" Biomedical Microdevices 3:323-330; Liu et al., 2003, "Solving the "world-to-chip" interface problem with a microfluidic matrix" Analytical Chemistry 75, 4718-23, Hong et al, 2004, "A nanoliter-scale nucleic acid processor with parallel architecture" Nature Biotechnology 22:435-39.

According to certain embodiments describer herein, the detection and/or quantification of one or more target nucleic acids from one or more samples may generally be carried out on a microfluidic device by obtaining a sample, optionally pre-amplifying the sample, and distributing the optionally pre-amplified sample, or aliquots thereof, into reaction chambers of a microfluidic device containing the appropriate buffers, primers, optional probe(s), and enzyme(s), subjecting these mixtures to amplification, and querying the aliquots for the presence of amplified target nucleic acids. The sample aliquots may have a volume of less than 1 picoliter or, in various embodiments, in the range of about 1 picoliter to about 500 nanoliters, in a range of about 2 picoliters to about 50 picoliters, in a range of about 5 picoliters to about 25 picoliters, in the range of about 100 picoliters to about 20 nanoliters, in the range of about 1 nanoliter to about 20 nanoliters, and in the range of about 5 nanoliters to about 15 nanoliters. In many embodiments, sample aliquots account for the majority of the volume of the amplification mixtures. Thus, amplification mixtures can have a volume of less than 1 picoliter or, in various embodiments about 2, about 5 about 7, about 10, about 15, about 20, about 25, about 50, about 100, about 250, about 500, and about 750 picoliters; or about 1, about 2, about 5, about 7, about 15, about 20, about 25, about 50, about 250, and about 500 nanoliters. The amplification mixtures can also have a volume within any range bounded by any of these values (e.g., about 2 picoliters to about 50 picoliters).

In certain embodiments, multiplex detection is carried out in individual amplification mixture, e.g., in individual reaction chambers of a microfluidic device, which can be used to further increase the number of samples and/or targets that can be analyzed in a single assay or to carry out comparative methods, such as comparative genomic hybridization (CGH). In various embodiments, up to 2, 3, 4, 5, 6, 7, 8, 9, 10, 50, 100, 500, 1000, 5000, 10000 or more amplification reactions are carried out in each individual reaction chamber.

In specific embodiments, the assay usually has a dynamic range of at least 3 orders of magnitude, more often at least 4, at least 5, at least 6, at least 7, or at least 8 orders of magnitude.

### Data Output and Analysis

In certain embodiments, when the methods of the invention are carried out on a matrix-type microfluidic device, the data can be output as a heat matrix (also termed "heat map"). In the heat matrix, each square, representing a reaction chamber on the Dynamic Array™ IFC matrix, has been assigned a color value which can be shown in gray scale, but is more typically shown in color. In gray scale, black squares indicate that no amplification product was detected, whereas white squares indicate the highest level of amplification produce, with shades of gray indicating levels of amplification product in between. In a further aspect, a software program may be used to compile the data generated in the heat matrix into a more reader-friendly format.

### Applications

The methods of the invention are applicable to any technique aimed at detecting the presence or amount of one or more target nucleic acids in a nucleic acid sample. Thus, for example, these methods are applicable to identifying the presence of particular polymorphisms (such as SNPs), alleles, or haplotypes, or chromosomal abnormalities, such as amplifications, deletions, or aneuploidy. The methods may be employed in genotyping, which can be carried out in a number of contexts, including diagnosis of genetic diseases or disorders, pharmacogenomics (personalized medicine), quality control in agriculture (e.g., for seeds or livestock), the study and management of populations of plants or animals (e.g., in aquaculture or fisheries management or in the determination of population diversity), or paternity or forensic identifications. The methods of the invention can be applied in the identification of sequences indicative of particular conditions or organisms in biological or environmental samples. For example, the methods can be used in assays to identify pathogens, such as viruses, bacteria, and fungi). The methods can also be used in studies aimed at characterizing environments or microenvironments, e.g., characterizing the microbial species in the human gut.

These methods can also be employed in determinations DNA or RNA copy number. Determinations of aberrant DNA copy number in genomic DNA is useful, for example, in the diagnosis and/or prognosis of genetic defects and diseases, such as cancer. Determination of RNA "copy number," i.e., expression level is useful for expression monitoring of genes of interest, e.g., in different individuals, tissues, or cells under different conditions (e.g., different external stimuli or disease states) and/or at different developmental stages.

In addition, the methods can be employed to prepare nucleic acid samples for further analysis, such as, e.g., DNA sequencing.

Finally, nucleic acid samples can be tagged as a first step, prior subsequent analysis, to reduce the risk that mislabeling or cross-contamination of samples will compromise the results. For example, any physician's office, laboratory, or hospital could tag samples immediately after collection, and the tags could be confirmed at the time of analysis. Similarly, samples containing nucleic acids collected at a crime scene could be tagged as soon as practicable, to ensure that the samples could not be mislabeled or tampered with. Detection of the tag upon each transfer of the sample from one party to another could be used to establish chain of custody of the sample.

### Kits

Kits according to the invention include one or more reagents useful for practicing one or more assay methods of the invention. A kit generally includes a package with one or more containers holding the reagent(s) (e.g., primers and/or probe(s)), as one or more separate compositions or, optionally, as admixture where the compatibility of the reagents will allow. The kit can also include other material(s) that may be desirable from a user standpoint, such as a buffer(s), a diluent(s), a standard(s), and/or any other material useful in sample processing, washing, or conducting any other step of the assay.

Kits according to the invention generally include instructions for carrying out one or more of the methods of the invention. Instructions included in kits of the invention can be affixed to packaging material or can be included as a package insert. While the instructions are typically written or printed materials they are not limited to such. Any medium capable of storing such instructions and communicating them to an end user is contemplated by this invention. Such media include, but are not limited to, electronic storage media (e.g., magnetic discs, tapes, cartridges, chips), optical media (e.g., CD ROM), RF tags, and the like. As used herein, the term "instructions" can include the address of an internet site that provides the instructions.

### EXAMPLES

### Example 1

### Selective enrichment of low molecular weight, apoptotic DNA from body fluids or other biological sources

### Problem# 1:

In pregnancy, cell free fetal DNA (CFF DNA) contributes a small proportion (~ 5%) of total circulating DNA found in plasma. The high maternal DNA background makes the determination of fetal aneuploidy difficult if not impossible.

CFF DNA has a length typically less than < 300 nucleotides (i.e. one or 2 nucleosomal lengths), whereas maternal cell-free DNA is present in nucleosome and sub-genome-length species, i.e. fetal cell-free DNA is typically smaller than a large proportion of maternal DNA.

Prior investigations have shown that maternal DNA can be depleted from circulating DNA by laborious gel electrophoresis recovery methods, however, these methods are limited by the intrinsic low recovery, low throughput and the laborious nature of such exercises.

Recent reports have demonstrated that digital PCR can be used for the detection of aneuploidy, even if only fractional amounts of the target DNA are derived from aneuploid cells.

Detection of fetal aneuploidy from maternal plasma samples via digital PCR applications such as the Fluidigm DID chip are limited by the relatively small amount of circulating fetal DNA compared to circulating total DNAs from maternal sources.

Here, is demonstrated preferential isolation and rapid enrichment of short DNA fragments from larger DNA. This approach can be adapted for preferential enrichment of short DNA from plasma by using PEG 8000 (Poly-ethylene glycol). Genome-length, high MW DNA is selectively precipitated, while short apoptotic-length DNA is retained in the supernatant for ease and practicality of collection. Fragmented DNA longer than 2 nucleosomes can be precipitated or left in the supernatant by adjusting the concentration of PEG 8000 and NaCl.

### Problem # 2 Description of a novel apoptotic DNA isolation method aiming to enhance Digital-PCR assay sensitivity:

It is known that mutations in the k-*ras* gene trigger the apoptosis pathway. Moreover, mutated k-*ras* DNA in serum is fragmented to nucleosome-length DNA. Evidence has accumulated that PCR-based mutation detection of k*-ras DNA* is highly dependent of the isolation method used, i.e., small DNA enrichment markedly improved mutation detection.

Addition of NaCl to 0.5 M and PEG 8000 to 8% specifically isolates apoptotic-length DNAs. The method described can be used to enhance detection of DNA (not only Trisomy 21 products, but colorectal cancer apopototic DNA, viral breakdown products, pre-neoplastic and malignant nucleic acid breakdown products), i.e., the method can enhance PCR assay sensitivity in situations that are accompanied by need to detect apoptotic or nucleosome-length nucleic acid. To the best of our knowledge, this approach has not been used for PCR enrichment methods and almost certainly, not for digital PCR applications.

Examining apoptotic-length, rather than genome-length, DNA in serum or plasma will increase the detection sensitivity of our Digital PCR "needle in a haystack" type approach and that apoptotic-length DNA can be can selectively isolated from genomic-length DNAs directly from serum.

Raw plasma, plasma lysate, or DNA extracted from plasma is mixed with approximately 1-5% or 1-8% PEG 8000 (MW 7000 - 9000) at approximately 1 M NaCl (or other monovalent salts). If necessary the sample will be incubated at 4 °C / on ice (at -20 °C) for 1 h or longer (overnight).

The sample will be centrifuged (approx 1500 g (but can be much higher) for 1-60 minutes, exact settings subject to optimization).

The supernatant is removed. Short nucleic acids are in the supernatant. Larger molecular weight species are localized to the pellet. The length cutoff depends on the conditions, namely PEG 8000 concentration.

If necessary nucleic acids are further extracted / purified through appropriate protocol, prior to further manipulation.

### Further developments:

Application to early detection/screening of cancer (circulating, apopototic - type tumor DNA has been reported to be shorter in some cases). Detection of increased apopototic DNA in subcellular or specific organs may indicate the presence of active apopotosis.

Large DNA fragments may be enriched with the same method.

Application to other body fluids.

Separation of cell-compartmented nucleic acids (nucleosomes, mitochondria. chloroplasts) from their milieu. Determination of small RNA localization within cells.

Useful for separation of small RNAs such as microRNA-length species from other nucleic acids. Enrichment of such species markedly increases detection of mature active miRNA and siRNA species, by reducing background large MW RNA contamination.

Other methods for selective isolation. For example, using different amounts of binding buffer with silica spin columns should also work. This method has been used to get rid of small and single-stranded nucleic acids, but not to collect the flow-through for the enrichment of body fluid and other short nucleic acid species

### Demonstration of Principle:

High molecular weight DNA (here a surrogate 2 log DNA marker) is selectively precipitated using PEG 8000 and NaCl and following centrifugation small nucleic acid-apopototic-length species are present in the supernatant.

### Method:

Low molecular weight DNA was isolated by mixing equal volumes of marker DNA with 20% PEG 8000 and adding NaCl to 0.5 M. Samples were incubated on ice for 30 min and centrifuged at 10,000x g for 20 min at Room Temp. Supernatant containing Low molecular weight DNA was collected and then fractionated in a 2% agarose gel and stained with ethidium bromide. Two different NaCl concentrations (0.5 and 1 M) were titrated in solutions also containing: 2.7%, 8.1%, 10.8% and 13 % PEG 8000. The default optimum solution is estimated to contain ~5% PEG and 0.5 M NaCl.

### Result:

The results are shown in Figure 2. Lane 2 shows the default solution, containing 5% PEG and 0.5 M NaCl centrifuged as described and fractionated by 2% agarose electrophoresis. Lane 2 demonstrates loss of high MW DNA, but selective retention of apoptotic-length DNAs in the supernatant. A control containing the same DNA sample, 0.5 M NaCl and 5% PEG but not centrifuged, providing a reference for electrophoretic mobility shift due to 0.5 M NaCl in the sample, is shown lane 12. Lane 1 contains the same DNA sample but completely untreated.

### Conclusion:

A 5% PEG and 0.5 M NaCl solution centrifuged at 10,000x g for 20 min selectively enriches for apoptotic-length DNA.

### Literature:

Quantitation of DNA fragmentation in apoptosis. Ioannou YA, Chen FW. Nucleic Acids Res. 1996 Mar 1;24(5):992-3.

Digital PCR for the molecular detection of fetal chromosomal aneuploidy.

Lo YM, Lun FM, Chan KC, Tsui NB, Chong KC, Lau TK, Leung TY, Zee BC, Cantor CR, Chiu RW. Proc Natl Acad Sci USA. 2007 Aug 7;104(32):13116-21. Epub 2007 Jul 30.

Detection of aneuploidy with digital polymerase chain reaction. Anal Chem. 2007 Oct 1;79(19):7576-9. Epub 2007 Aug 24. Fan HC, Quake SR.

Preferential Isolation of Fragmented DNA Enhances the Detection of Circulating Mutated k-ras DNA, Mengjun Wang, 1 Timothy M. Block, Laura Steel, Dean E. Brenner, and Ying-Hsiu Su1, Clinical Chemistry 2004, 50, 211-213, No. 1.

## Claims

1. A method of enriching a sample for short nucleic acids of less than 300 nucleotides in length, the method comprising:
circularizing the sample nucleic acids under conditions that favor the circularization of short nucleic acids; and
recovering the circularized nucleic acids.

2. The method of claim 1 wherein said circularizing is carried out by contacting the sample nucleic acids with a circligase.

3. The method of any of the preceding claims, additionally comprising nucleic acid amplification and/or DNA sequencing to detect and/or quantify target nucleic acids within the recovered nucleic acids.

4. The method of any of the preceding claims, wherein the sample is unfractionated prior to enrichment.

5. The method of any of the preceding claims, wherein the sample comprises a sample of a bodily fluid, or a fraction thereof, from a cancer patient.

6. The method of any of the preceding claims, wherein the sample comprises a sample of a maternal bodily fluid, or a fraction thereof, from a pregnant subject.

7. The method of claim 6, wherein at least some of the recovered nucleic acids comprise fetal DNA.

8. The method of claim 6, wherein the maternal bodily fluid is selected from the group consisting of whole blood, plasma, urine, and cervico-vaginal secretions.

9. The method of claim 6, wherein the method comprises determining a fetal genotype.

10. The method of claim 6, wherein the method comprises detecting the presence of a mutation or fetal aneuploidy.

## Patentansprüche

1. Verfahren zum Anreichern einer Probe für kurze Nukleinsäuren mit einer Länge von weniger als 300 Nukleotiden, wobei das Verfahren folgendes umfasst:
Zirkulieren der Probennukleinsäuren unter Bedingungen, welche die Zirkulation kurzer Nukleinsäuren begünstigen; und
Rückgewinnen der zirkulierten Nukleinsäuren.

2. Verfahren nach Anspruch 1, wobei das Zirkulieren ausgeführt wird durch Inkontaktbringen der Probennukleinsäuren mit einer Circligase.

3. Verfahren nach einem der vorstehenden Ansprüche, zusätzlich umfassend Nukleinsäureamplifikation und/oder DNA-Sequenzierung zum Nachweisen und/oder Quantifizieren von Zielnukleinsäuren in den rückgewonnenen Nukleinsäuren.

4. Verfahren nach einem der vorstehenden Ansprüche, wobei die Probe vor der Anreicherung unfraktioniert wird.

5. Verfahren nach einem der vorstehenden Ansprüche, wobei die Probe eine Probe eines Körperfluids oder eines Teils davon von einem Krebspatienten umfasst.

6. Verfahren nach einem der vorstehenden Ansprüche, wobei die Probe eine Probe eines maternalen Körperfluids oder eines Teils davon von einem schwangeren Subjekt umfasst.

7. Verfahren nach Anspruch 6, wobei wenigstens ein Teil der rückgewonnenen Nukleinsäuren fötale DNA umfasst.

8. Verfahren nach Anspruch 6, wobei das maternale Körperfluid ausgewählt ist aus der Gruppe bestehend aus Vollblut, Plasma, Urin und cervico-vaginale Sekretionen.

9. Verfahren nach Anspruch 6, wobei das Verfahren das Bestimmen eines fötalen Genotyps umfasst.

10. Verfahren nach Anspruch 6, wobei das Verfahren das Nachweisen des Vorhandenseins einer Mutation oder fötaler Aneuploidie.

## Revendications

1. Procédé permettant d'enrichir un échantillon en acides nucléiques courts de moins de 300 nucléotides de long, le procédé comprenant les étapes consistant à :
circulariser les acides nucléiques de l'échantillon dans des conditions qui favorisent la circularisation des acides nucléiques courts ; et
récupérer les acides nucléiques circularisés.

2. Procédé selon la revendication 1, ladite circularisation étant effectuée en mettant en contact les acides nucléiques de l'échantillon avec une circligase.

3. Procédé selon l'une quelconque des revendications précédentes, comprenant en outre l'amplification des acides nucléiques et/ou le séquençage de l'ADN pour détecter et/ou quantifier les acides nucléiques cibles dans les acides nucléiques récupérés.

4. Procédé selon l'une quelconque des revendications précédentes, l'échantillon étant non fractionné avant l'enrichissement.

5. Procédé selon l'une quelconque des revendications précédentes, l'échantillon comprenant un échantillon d'un fluide corporel, ou d'une fraction de celui-ci, d'un patient atteint de cancer.

6. Procédé selon l'une quelconque des revendications précédentes, l'échantillon comprenant un échantillon d'un fluide corporel maternel, ou une fraction de celui-ci, d'un sujet enceinte.

7. Procédé selon la revendication 6, au moins certains des acides nucléiques récupérés comprenant de l'ADN foetal.

8. Procédé selon la revendication 6, le fluide corporel maternel étant choisi dans le groupe constitué de sang total, de plasma, d'urine et de sécrétions cervico-vaginales.

9. Procédé selon la revendication 6, le procédé comprenant l'étape consistant à déterminer un génotype foetal.

10. Procédé selon la revendication 6, le procédé comprenant l'étape consistant à détecter la présence d'une mutation ou d'aneuploïdie foetale.
